# EUROPÄISCHE PATENTSCHRIFT

(11) **EP 2 582 407 B1**
(45) Veröffentlichungstag und Bekanntmachung des Hinweises auf die Patenterteilung: **27.08.2014**
(21) Anmeldenummer: 11725741.0
(22) Anmeldetag: 15.06.2011
(51) Int. Cl.: A61L 27/30, A61F 2/28, A61L 24/02, A61L 27/04

(54) **KNOCHENIMPLANTAT, ENTHALTEND EINEN MAGNESIUMHALTIGEN METALLISCHEN WERKSTOFF MIT VERMINDERTER KORROSIONSRATE UND VERFAHREN UND SET ZU DESSEN HERSTELLUNG**
BONE IMPLANT COMPRISING A MAGNESIUM-CONTAINING METALLIC MATERIAL WITH REDUCED CORROSION RATE, AND METHODS AND KIT FOR PRODUCING THE BONE IMPLANT
IMPLANT OSSEUX COMPRENANT UN MATÉRIAU MÉTALLIQUE CONTENANT DU MAGNÉSIUM AVEC UN TAUX DE CORROSION RÉDUIT, ET PROCÉDÉ ET ENSEMBLE POUR LA FABRICATION DUDIT IMPLANT OSSEUX

(30) Priorität: 15.06.2010 DE 102010030135
(43) Veröffentlichungstag der Anmeldung: 24.04.2013
(73) Patentinhaber: InnoTERE GmbH, 01445 Radebeul (DE)
(72) Erfinder: NIES, Berthold, 64407 Fränkisch-Crumbach (DE); GLORIUS, Stefan, 78462 Konstanz (DE)
(74) Vertreter: Kailuweit & Uhlemann Patentanwälte
(86) Internationale Anmeldenummer: PCT/EP2011/059954
(87) Internationale Veröffentlichungsnummer: WO 2011/157758

(56) Entgegenhaltungen:
- WO-A1-02/02478
- DE-A1-102008 010 210
- US-A1- 2009 306 725

## Beschreibung

Die Erfindung betrifft ein Knochenimplantat, welches einen magnesiumhaltigen metallischen Werkstoff mit verminderter Korrosionsrate und mineralischen Knochenzement enthält sowie Verfahren und ein Set zu dessen Herstellung.

Häufig ist bei der Verwendung von medizinischen Implantaten nur ein zeitweiliger Verbleib des Implantatmaterials im Körper erforderlich. Um bei derartigen Implantationen auf eine aufwendige Operation zur Entfernung von Implantatmaterialien, die vom Körper nicht abgebaut werden können, zukünftig zu verzichten, wird seit vielen Jahren an der Entwicklung bioresorbierbarer Implantatmaterialien geforscht. Bioresorbierbare Materialien besitzen die Eigenschaft, nach der Implantation im Körper allmählich abgebaut zu werden. Die dabei entstehenden Abbauprodukte werden vom Körper weitgehend resorbiert.

Der Abbau von Implantatmaterialien kann aktiv oder passiv erfolgen. Beim aktiven Abbau durch den Organismus wird das Implantatmaterial durch enzymatische oder zelluläre Mechanismen degradiert. Dies geschieht beispielsweise bei Implantatmaterialien aus Kollagen oder mineralischen Knochenzementen auf der Basis von Calciumphosphaten. Der aktive Abbau von Implantatmaterialien ist besonders dann gewünscht, wenn der Abbau im Rahmen des natürlichen Stoffwechsels erfolgt und nicht auf einer Entzündungsreaktion beruht.

Ein passiver Abbau erfolgt bei Implantatmaterialien, die in der biologischen Umgebung nicht stabil sind. Dieser passive Abbau wird hierin als Biodegradation bezeichnet. Er ist typisch für die resorbierbaren Polymere, aus denen z. B. resorbierbare Nahtmaterialien hergestellt werden. Ebenfalls passiv erfolgt der Abbau von Metallen, die im biologischen Milieu nicht stabil sind. Dies wird hierin auch als Korrosion bezeichnet. Unter Korrosion ist dabei die Umwandlung metallischer Materialien in ihre Oxide oder Salze, also in mineralische Stoffe, zu verstehen. Für die metallischen Implantatmaterialien hat sich der Begriff der Biokorrodierbarkeit, also der Korrosion im biologischen Milieu etabliert. Üblicherweise wird der Begriff für die erwünschte Korrosion eines metallischen Implantats im Körper verwendet. Als biokorrodierbare Implantatmetalle gelten insbesondere Magnesium und dessen Legierungen sowie Eisen und dessen unedle Legierungen. Vor allem die magnesiumhaltigen metallischen Werkstoffe finden vermehrt großes Interesse, weil sie deutlich höhere Festigkeiten als resorbierbare Polymere aufweisen und weil Magnesium das einzige technisch verfügbare Metall ist, das im Körper in größeren Mengen vorkommt und das in Form seines Ions in zahlreiche biochemische Reaktionen eingebunden ist. Anders als bei Abbauprodukten von Eisen können Magnesiumionen aus den Korrosionsprodukten von magnesiumhaltigen Implantaten vom Körper leicht ausgeschieden werden.

Biokorrodierbare Implantate auf der Basis resorbierbarer Polymere sind seit geraumer Zeit auf dem Markt und haben in einigen Indikationen mit geringer mechanischer Belastung wirtschaftliche Bedeutung erlangt. Ihre weitere Verbreitung ist jedoch durch die geringe Bioverträglichkeit der Abbauprodukte und die wenig reproduzierbaren mechanischen Eigenschaften im Verlauf des Abbauprozesses limitiert.

Da besonders orthopädische Implantate hohe mechanische Anforderungen erfüllen müssen, wird seit einigen Jahren intensiv an Implantatmaterialien auf der Basis biokorrodierbarer Metalllegierungen gearbeitet. Magnesiumlegierungen sind für diese Anwendung grundsätzlich sehr gut geeignet, da bei deren Abbau vorwiegend Abbauprodukte in der Form von Magnesiumsalzen gebildet werden, die als natürliche Bestandteile des Stoffwechsels bioverträglich sind. In verschiedenen Studien sind Implantate aus Magnesiumlegierungen bereits für kardiovaskuläre Anwendungen und als Knochenimplantate untersucht worden. Dabei konnte in den meisten Fällen eine gute Verträglichkeit nachgewiesen werden.

Bei der Korrosion von Magnesium ist jedoch eine starke Gasbildung, vorwiegend Wasserstoff, feststellbar. Dies führte bei großvolumigen Knochenimplantaten zur Bildung von Gasblasen im Gewebe, da der Abtransport nicht in ausreichendem Maße sichergestellt war. Die Korrosion erfolgt einerseits zu schnell und andererseits unkontrolliert, also nicht gleichmäßig von außen nach innen, sondern häufig punktuell konzentriert. Eine hinreichend zuverlässige Einstellung von mechanischen Eigenschaften eines Implantats über die Zeit ist so nicht möglich. Daher wird an der Entwicklung von Magnesiumimplantaten gearbeitet, deren Korrosion in verringertem Maße voranschreitet. Die bisher zu schnelle und unkontrollierbare Korrosion von Magnesiumwerkstoffen soll durch verschiedene Modifikationen kontrollierbar gestaltet werden.

Bisher werden dazu zwei verschiedene Ansätze verfolgt. Zum einen werden verschiedene Magnesiumlegierungen erprobt. Zum anderen werden Magnesium und Magnesiumlegierungen mit Beschichtungen versehen, die die Korrosion des Metalls hemmen sollen.

Bisher bekannte Beschichtungen zur Korrosionshemmung von magnesiumbasierten Implantaten beruhen auf dem Aufbringen polymerer oder anorganischer Schichten, die chemische Umwandlung der Metalloberfläche, Heißgasoxidation, Anodisieren, Plasmaspray-Verfahren, Lackieren oder ähnliche Verfahren.

DE 101 63 106 A1 offenbart Magnesiumwerkstoffe, bei denen die Korrosionseigenschaften durch das Zulegieren von Halogeniden verändert werden. Nachteilig ist hierbei, dass durch das Zulegieren auch die mechanischen Eigenschaften des Werkstoffs und nicht nur dessen Korrosionseigenschaften verändert werden.

US 2009/0306725 offenbart ein aus Mg-Materialien bestehendes Knochenimplantat, das über eine Antikorrosionsbeschichtung verfügt, welche Phosphor- und Calciumionen enthält. Die aufzutragende Filmlösung enthält zudem Al-Mg-Mischoxide.

DE 10 2006 060 501 A1 offenbart Verfahren zur Herstellung einer korrosionshemmenden Schicht auf Magnesiumlegierungen. Dabei wird eine biokorrodierbare Magnesiumlegierung mit Hilfe einer anodischen plasmachemischen Behandlung in einem wässrigen Elektrolyten beschichtet. Der Elektrolyt enthält dabei in einer Ausführungsform mindestens NH₃, H₃PO₄ und H₃BO₃. In einer anderen Ausführungsform enthält der Elektrolyt NaMnO₄ und NH₄VO₃. Im Verfahren wird der Magnesiumwerkstoff als Anode geschaltet. Die Anodisation erfolgt über Plasmaentladungen im Elektrolyten an der Oberfläche des zu beschichtenden Werkstoffteils.

DE 10 2008 043 970 A1 offenbart Verfahren zur Herstellung von korrosionshemmenden Beschichtungen auf Magnesiumwerkstoffen, in denen die Werkstoffe in einer wässrigen oder alkoholischen Konversionslösung behandelt werden und einer anodischen Oxidation unterzogen werden. In der Konversionslösung sind Ionen in einer Konzentration von 10⁻² mol/l bis 2 mol/l enthalten. Die Ionen sind dabei ausgewählt aus mindestens einem der Gruppe K⁺, Na⁺, NH₄⁺, Ca²⁺, Mg²⁺, Zn²⁺, Ti⁴⁺, Zr⁴⁺, Ce³⁺, PO₃³⁻, PO₄³⁻, HPO₄²⁻, H₂PO⁴⁻, OH⁻, BO₃³⁻, B₄O₇³⁻, SiO₃²⁻, MnO₄²⁻, MnO₄⁻, VO₃⁻, WO₄²⁻, MoO₄²⁻, TiO₃²⁻, Se²⁻, ZrO₃²⁻ und NbO₄⁻.

Trotz der umfangreichen Arbeiten zur Entwicklung neuer Magnesiumlegierungen wurden weder für die kardiovaskuläre Anwendung als Stents, noch für die Verwendung als Knochenimplantate bisher Lösungen gefunden, die Korrosionsrate auf ein ausreichend niedriges Niveau zu senken, um die mechanische Funktion der Implantate für den gewünschten Zeitraum von > 6 Wochen für die kardiovaskuläre Anwendung bzw. auch längeren Zeiträumen von > 6 Monaten für die Anwendung als Knochenimplantat gewährleisten zu können.

Daher besteht nach wie vor Bedarf an alternativen Ansätzen zu Kontrolle der Korrosion von Implantaten auf Magnesiumbasis.

Aufgabe der Erfindung ist es, Knochenimplantate mit magnesiumhaltigen metallischen Werkstoffen bereitzustellen, die eine hohe Korrosionsbeständigkeit und verbesserte mechanische Eigenschaften aufweisen und Verfahren und Mittel zu deren Herstellung anzugeben.

Die Aufgabe wird erfindungsgemäß gelöst durch ein Verfahren zur Herstellung eines Knochenimplantats, welches einen magnesiumhaltigen metallischen Werkstoff mit einer Magnesium-Ammonium-Phosphate enthaltenden korrosionshemmenden Beschichtung und mineralischen Knochenzement umfasst. Nach dem erfindungsgemäßen Verfahren wird ein magnesiumhaltiger metallischer Werkstoff, dessen Oberfläche eine Magnesiumoxidschicht und/oder eine Magnesiumsalzschicht aufweist, zur Erzeugung eines festen Verbundmaterials, welches den mineralischen Knochenzement und den magnesiumhaltigen metallischen Werkstoff enthält, mit mineralischem Knochenzement kombiniert. Der mineralische Knochenzement enthält dabei mineralische Pulverbestandteile, die in Gegenwart von Wasser zu einem Feststoff abbinden und wasserlösliche phosphationenhaltige Salze, vorzugsweise wasserlösliche phosphationenhaltige und ammoniumionenhaltige Salze. Erfindungsgemäß wird der magnesiumhaltige metallische Werkstoff vor und/oder während der Kombination mit dem mineralischen Knochenzement in Gegenwart wasserlöslicher ammoniumionen- und phosphationenhaltiger Salze (vorzugsweise Ammoniumphosphate) mit Wasser in Kontakt gebracht. Weiterhin wird der mineralische Knochenzement zur Abbindung mit Wasser in Kontakt gebracht. Das zur Erzeugung der korrosionshemmenden Beschichtung notwendige Wasser und das zur Auslösung der Abbindereaktion des mineralischen Knochenzements erforderliche Wasser liegt in Form von reinem Wasser oder als wässrige Zubereitung (vorzugsweise wässrige Lösung) vor. Dabei ist der Einsatz von wässrigen Zubereitungen gegenüber reinem Wasser bevorzugt.

Die Erfindung umfasst auch ein Knochenimplantat, welches vorzugsweise durch ein erfindungsgemäßes Verfahren erhältlich ist. Das erfindungsgemäße Knochenimplantat umfasst einen magnesiumhaltigen metallischen Werkstoff und mineralischen Knochenzement, der wasserlösliche phosphationenhaltige Salze enthält. Dabei weist der magnesiumhaltige metallische Werkstoff erfindungsgemäß eine Beschichtung auf, die Magnesium-Ammonium-Phosphate enthält (Passivierungsschicht).

Von der Erfindung umfasst ist auch ein Set zur Herstellung eines erfindungsgemäßen Knochenimplantats. Das erfindungsgemäße Set enthält
a) eine Knochenzementzubereitung umfassend mineralisches Knochenzementpulver und wasserlösliche phosphationenhaltige Salze, und
b) einen magnesiumhaltigen metallischen Werkstoff mit einer Magnesiumoxidschicht und/oder einer Magnesiumsalzschicht und wasserlösliche ammonium- und phosphationenhaltige Salze, vorzugsweise Ammoniumphosphate, oder
c) einen magnesiumhaltigen metallischen Werkstoff mit einer Magnesium-Ammonium-Phosphate enthaltenden Beschichtung und ggf. wasserlösliche ammonium- und phosphationenhaltige Salze, vorzugsweise Ammoniumphosphate.

Die Erfindung beruht auf der Entdeckung der Erfinder, dass Magnesium und Magnesiumlegierungen durch eine Behandlung mit Ammoniumphosphaten in wässrigen Lösungen gegen Korrosion geschützt werden. Bei Beschädigung der Magnesium-Ammonium-Phosphat-Schicht auf dem magnesiumhaltigen metallischen Werkstoff kann die darunter liegende Schicht, die Magnesiumoxide und/oder Magnesiumsalze enthält (hierin auch als Konversionsschicht bezeichnet), zu Magnesium-Ammonium-Phosphat unter Umsetzung der im mineralischen Knochenzement enthaltenen Phosphationen umgewandelt werden, so dass die korrosionshemmende Schicht erneuert wird. Dies trifft auch zu, wenn die Passivierungsschicht bis auf den metallischen Untergrund abgetragen wird, da sich in wässriger Umgebung, insbesondere auch in Körperflüssigkeit, die Oberfläche des magnesiumhaltigen Metalls spontan wieder mit einer Salzschicht (insbesondere Mg(OH)₂ oder Magnesium-Hydroxid-Carbonat) überzieht. Durch die Kombination des mineralischen Knochenzements mit dem metallischen Werkstoff werden gleichzeitig die mechanischen Eigenschaften des erhaltenen Knochenimplantats verbessert, da diese durch die geeignete Wahl von mineralischen Knochenzementen einstellbar und an die entsprechenden Bedürfnisse der jeweiligen Implantation anpassbar sind.

Zur Bereitstellung der korrosionshemmenden Beschichtung auf der Oberfläche des magnesiumhaltigen metallischen Werkstoffs ist es notwendig, diesen während der Herstellung des Knochenimplantats mit einer wässrigen, ammonium- und phosphationenhaltigen Zubereitung in Kontakt zu bringen. Durch die Kontaktierung des magnesiumhaltigen metallischen Werkstoffes (hierin mitunter auch vereinfacht als "Werkstoff" bezeichnet) mit wässrigen Lösungen, welche Ammoniumionen und Phosphationen enthalten, bildet sich auf der Oberfläche des Werkstoffs eine Magnesium-Ammonium-Phosphate, insbesondere Mg(NH₄)PO₄ x 6 H₂O, enthaltende Schicht (hierin auch als Passivierungsschicht bezeichnet). Die Magnesium-Ammonium-Phosphate verlangsamen die Korrosion des magnesiumhaltigen metallischen Werkstoffs (Passivierung). Die Magnesium-Ammonium-Phosphat-Schicht auf der Oberfläche des metallischen magnesiumhaltigen Werkstoffs ist nur gering wasserdurchlässig. Dadurch wird der Zutritt von Wasser zum metallischen Magnesium erschwert und verlangsamt, so dass der metallische Werkstoff langsamer korrodiert (korrosionshemmende Beschichtung). Es erfolgt also noch eine Korrosion des magnesiumhaltigen metallischen Werkstoffes, allerdings mit reduzierter Geschwindigkeit.

Die Anwesenheit der wasserlöslichen phosphationenhaltigen Salze im mineralischen Knochenzement unterstützt die Erneuerung der korrosionshemmenden Beschichtung. Dies wird in Gegenwart von mineralischen Knochenzementen, die sowohl wasserlösliche phosphationen- als auch ammoniumionenhaltige Salze (insbesondere Ammoniumphosphate) enthalten, nochmals verbessert. Daher sind mineralische Knochenzemente bevorzugt, die sowohl wasserlösliche phosphationenhaltige Salze als auch wasserlösliche ammoniumionenhaltige Salze enthalten. Die Anwesenheit von phosphationen- und ammoniumionenhaltige Salzen im mineralischen Knochenzement trägt weiterhin vorteilhaft zur Erhöhung der Druckfestigkeit des Knochenimplantats bei.

Als magnesiumhaltige metallische Werkstoffe im Sinne der Erfindung gelten alle Metalle und Metalllegierungen, die Magnesium zu einem Anteil von mindestens 25 Gewichts-%, vorzugsweise mindestens 50 Gewichts-%, bevorzugt mindestens 75 Gewichts-% enthalten.

Ein besonders bevorzugter Werkstoff ist Magnesium mit einer Reinheit von mindestens 99 Gew.-% (Reinmagnesium). Weiter bevorzugte Werkstoffe sind Magnesiumlegierungen, insbesondere Legierungen von Magnesium, die die Legierungsmetalle Aluminium (A), Wismut (B), Kupfer (C), Seltene Erden (E), Eisen (F), Zirkon, (K), Lithium (L), Mangan (M), Silber (Q), Silizium (S), Yttrium (W), Zink (Z) und Calcium allein oder in Kombination umfassen. Bevorzugt sind Mehrfachlegierungen, also Legierungen von Magnesium mit mindestens zwei Legierungspartnern, besonders bevorzugt sind Mehrfachlegierungen, die Yttrium, Seltene Erden, Mangan, Zink oder Zirkon allein oder in Kombination enthalten. Eine bevorzugte Legierung ist die WE43, also eine Magnesium-Legierung, die Yttrium und Seltene Erden enthält. Besonders bevorzugt sind Magnesium-Legierungen, die im Vergleich zu Reinmagnesium bereits eine erhöhte Korrosionsdauer aufweisen, insbesondere Legierungen, die mindestens Magnesium und Silizium und ggf. weitere Legierungspartner enthalten.

Der magnesiumhaltige metallische Werkstoff kann für den Einsatz in der Erfindung beliebige Geometrien aufweisen. Bevorzugt sind Werkstoffe in der Form von Fasern, Fäden, Filamenten, Drähten. Davon bevorzugt sind Fasern, vorzugsweise mit einer Dicke von 10-1000 µm und einer Länge von 1-100 mm (Kurzfasern). Weiter bevorzugt sind Werkstoffe in Form von zu Geweben, Geflechten, Gewirken, Gestricken, Vliesen, Vliesstoffen, Membranen oder Kordeln verarbeiteten Drähten oder Fäden. Weiter bevorzugt sind Werkstoffe aus aus Fasern, Fäden, Filamenten oder Drähten erhältlichen Flächengebilden, vorzugsweise durch Verpressen und/oder Sintern von Fasern, Fäden, Filamenten oder Drähten erhältlichen Flächengebilden. Weiter bevorzugt sind Werkstoffe in Form eines offenzelligen Metallschaums. Eine Form offenzelliger Metallschäume sind Formkörper, die aus perforierten flächigen Metallstrukturen, vorzugsweise aus perforierten Blechen oder Streckmetall, aufgebaut sind. Beim Übereinanderschichten mehrerer Lagen entstehen dabei durchgehende Porensysteme. Die Verbindung der einzelnen Lagen erfolgt beispielsweise durch Verschweißen, Verkleben oder mechanische Verzahnung. Für die Erfindung besonders geeignete Formkörper sind stabförmige Formkörper, die durch Aufwickeln perforierter Bleche oder Streckmetalle mit anschließender Verbindung der einzelnen Lagen untereinander erhältlich sind. Weiter bevorzugt sind Werkstoffe in Form orthopädischer oder osteosynthetischer Implantate, insbesondere Platten, Schrauben, Nägel, Stifte oder Cages. Weiter bevorzugt sind Werkstoffe in partikulärer Form, vorzugsweise in Form von Spänen. Davon bevorzugt sind Partikel, insbesondere Späne, mit einer Größe (maximalen Ausdehnung) von 10 - 1000 µm. Magnesiumhaltige metallische Partikel haben den Vorteil, dass diese mit der Pulverkomponente des mineralischen Knochenzements homogen vermischt werden können, so dass ein Verbundmaterial mit gleichmäßig darin verteilten metallischen Partikeln erhalten werden kann.

Es werden in der Erfindung biokorrodierbare magnesiumhaltige metallische Werkstoffe eingesetzt, also Werkstoffe, die im Körper abgebaut werden. Im Falle der Verwendung einer magnesiumhaltigen Legierung sind vorzugsweise keine Metalle als Legierungspartner enthalten, deren Abbauprodukte (Korrosionsprodukte) biologisch unverträglich sind. Vorzugsweise sind keine Metalle als Legierungspartner enthalten, bei denen bei der Biokorrosion biologisch unverträgliche Konzentrationen des Metalls freigesetzt werden. Bevorzugt sind Legierungen von Magnesium mit Silber und/oder Kupfer. Vorteilhaft werden bei Biokorrosion dieser Legierungen Abbauprodukte gebildet, die antimikrobiell wirken.

Die Aufbringung der korrosionshemmenden Beschichtung auf den magnesiumhaltigen metallischen Werkstoff erfolgt nach der endgültigen Formgebung des metallischen Werkstoffs. Die Kombination des magnesiumhaltigen metallischen Werkstoffs mit dem mineralischem Knochenzement erfolgt entweder vor oder während der Implantation, bevorzugt vor der Implantation.

Die Bildung der Magnesium-Ammonium-Phosphate erfolgt durch die Umwandlung von Magnesiumsalzen und/oder Magnesiumoxiden, die sich auf der Oberfläche der magnesiumhaltigen metallischen Werkstoffe befinden. Bei der Lagerung magnesiumhaltiger metallischer Werkstoffe bilden sich auf deren Oberfläche spontan magnesiumhaltige Oxide und/oder Salzverbindungen, insbesondere Oxide, Hydroxide, Carbonate oder Hydroxid-Carbonate.

Diese Oxide oder Salzverbindungen reagieren mit Ammoniumionen und Phosphationen zu schwerlöslichen Magnesium-Ammonium-Phosphaten, insbesondere durch die folgenden Reaktionen:

(1) MgO + NH₄⁺ + H₂PO₄⁻ + 5 H₂O → Mg(NH₄)PO₄ x 6 H₂O

(2) Mg(OH)₂ + NH₄⁺ + H₂PO₄⁻ + 4 H₂O → Mg(NH₄)PO₄ x 6 H₂O

(3) MgCO₃ + NH₄⁺ + H₂PO₄⁻+ 6 H₂O → Mg(NH₄)PO₄ x 6 H₂O + H₂CO₃

(4) 4 MgCO₃ x Mg(OH)₂ x 4 H₂O + NH₄⁺ + H₂PO₄⁻ + n H₂O → 5 Mg(NH₄)PO₄ x 6 H₂O + 4 H₂CO₃

Bei der Erfindung werden zur Erzeugung der Magnesium-Ammonium-Phosphate enthaltenden korrosionshemmenden Beschichtung auf einem magnesiumhaltigen metallischen Werkstoff ammoniumionen- und phosphationenhaltige Salze eingesetzt. Ammoniumionenhaltige Salze und phosphationenhaltige Salze, die bei der Erfindung verwendet werden, sind wasserlöslich. Unter wasserlöslich wird im Sinne der Erfindung eine Löslichkeit in Wasser von mindestens 0,1 mol/l, bevorzugt 1,0 mol/l verstanden.

Bevorzugte wasserlösliche ammoniumionenhaltige Salze sind ausgewählt aus Ammoniumacetat, Ammoniumbicarbonat, Ammoniumcarbamat, Ammoniumcarbonat, mono-, di- und tribasischem

Ammoniumcitrat, Ammoniumfluorid, Ammoniumsalzen von Aminosäuren (insbesondere Ammoniumglutamat), Ammoniumhydroxid, Ammoniumsalzen von Fettsäuren (insbesondere Ammoniumoleat), Ammoniumsalzen von Anionentensiden (insbesondere Ammoniumlauryl-sulfat, Ammoniumcetyl-phosphat), Ammoniumlactat, Ammonium-Natrium-Phosphat, Ammonium-KaliumPhosphat, Ammoniumnitrat, Ammoniumsulfat und Ammoniumtartrat.

Bevorzugte wasserlösliche phosphationenhaltige Salze sind ausgewählt aus Natriumhydrogenphosphat, Tri-Natriumphosphat, Natrium-di-Hydrogenphosphat, Natrium-Glycerophosphat, Natrium-Pyrophosphaten, Natrium-Polyphosphaten, Kaliumhydrogenphosphat, Tri-Kaliumphosphat, Kaliumdihydrogenphosphat, Kalium-Glycerophosphat, Kalium-Pyrophosphaten und Kalium-Polyphosphaten.

Bevorzugte Kombinationen ammoniumionen- und phosphationenhaltiger Salze sind Ammoniumchlorid (NH₄Cl) und Natriumphosphat (NaH₂PO₄) oder Ammoniumsulfat (NH₄)₂SO₄ und Natriumphosphat (Na₂HPO₄).

Bevorzugt sind wasserlösliche Ammoniumphosphate. Unter Ammoniumphosphaten sind in diesem Zusammenhang alle Verbindungen zu verstehen, die sowohl Ammonium- als auch Phosphationen enthalten. Bevorzugte Ammoniumphosphate sind ausgewählt aus: (NH₄)₃PO₄, (NH₄)₂HPO₄, (NH₄)H₂PO₄, Ammoniumsalzen der di-, oligo- und polymeren Phosphorsäure, sowie der teilweise substituierten Phosphorsäure, insbesondere Ammonium-Glycerophosphat und deren Mischungen untereinander. Besonders bevorzugte Ammoniumphosphate sind ausgewählt aus (NH₄)₃PO₄, (NH₄)₂HPO₄ und (NH₄)H₂PO₄ sowie deren Kombinationen.

Der mineralische Knochenzement, der erfindungsgemäß eingesetzt wird, enthält wasserlösliche phosphationenhaltige Salze. Bevorzugte phosphationenhaltige Salze, die in dem mineralischen Knochenzement enthalten sind, sind ausgewählt aus Natriumhydrogenphosphat, Tri-Natriumphosphat, Natrium-di-Hydrogenphosphat, Natrium-Glycerophosphat, Natrium-Pyrphosphaten, Natrium-Polyphosphaten, Kaliumhydrogenphosphat, Tri-Kaliumphosphat, Kaliumdihydrogenphosphat, Kalium-Glycerophosphat, Kalium-Pyrophosphaten und Kalium-Polyphosphaten.

Bevorzugt sind im mineralischen Knochenzement ammonium- und phosphationen-haltige Salze, vorzugsweise wasserlösliche Ammoniumphosphate, insbesondere die oben definierten Ammoniumphosphate, enthalten. Die wasserlöslichen Salze (Phosphate, vorzugsweise ammonium- und phosphationenhaltigen Salze, bevorzugt Ammoniumphosphate) sind im mineralischen Knochenzement vorzugsweise in einer Gesamtkonzentration von 0,1 bis 30 Gew.-%, besonders bevorzugt 0,1 bis 10 Gewichts-%, ganz besonders bevorzugt von 0,1 bis 15 Gewichts-% enthalten. Mineralische Knochenzemente im erfindungsgemäßen Sinn sind alle Knochenzemente, die mineralische Bestandteile enthalten, die mit Wasser und/oder wässrigen Lösungen unter Bildung eines unlöslichen Feststoffs reagieren (hierin auch als "abbinden" bezeichnet). Bevorzugt sind hydraulisch abbindende mineralische Knochenzemente. Im Unterschied zu polymeren Knochenzementen, wie beispielsweise Polymethylmethacrylat-Knochenzementen (PMMA-Zement), ist zur Bildung des Feststoffs keine Polymerisation erforderlich. Der Nachteil polymerer Knochenzemente ist, dass diese nicht resorbierbar sind. Mineralische Knochenzemente können neben den mineralischen Bestandteilen polymere Zusätze enthalten, die sowohl die Mechanik, die Handhabungseigenschaften, die biologischen Eigenschaften, als auch die Reaktionskinetik beeinflussen können. Die Hauptkomponente des Feststoffs ist jedoch auch bei mineralischen Knochenzementen mit Polymerzusatz vorzugsweise mineralisch, d.h. mineralische Knochenzemente enthalten vorzugsweise maximal 50 Gewichts-% an Polymerzusätzen.

Mineralische Knochenzemente umfassen eine Pulverkomponente, welche zur Auslösung der Abbindereaktion mit einer wässrigen Flüssigkeit vermengt wird. Die Pulverkomponente enthält dabei die mit Wasser reaktiven festen Bestandteile. Durch die Reaktion der reaktiven festen Bestandteile der Pulverkomponente mit Wasser (Abbindereaktion) entsteht ein wasserunlöslicher Feststoff. Auch der abgebundene Feststoff wird im Sinne der Erfindung als "mineralischer Knochenzement" bezeichnet. Die Abbindereaktion des mineralischen Knochenzements kann vor, während oder nach der Kombination mit dem beschichteten magnesiumhaltigen metallischen Werkstoff ausgelöst werden.

Bevorzugte Bestandteile der Pulverkomponente des mineralischen Knochenzements, die bei Kontakt mit Wasser zu einem Feststoff abbinden sind Calciumphosphate und/oder Magnesiumphosphate, insbesondere α-TCP, β-TCP, tetra-Calciumphosphat, amorphes Calciumphosphat, Mono-Calciumphosphat, Calciumhydrogenphosphat, Mg₃(PO₄)₂, und/oder CaₓMg₃₋ₓ(PO₄)₂ (mit 0 < x < 3). Die Zusammensetzung mineralischer Knochenzemente ist dem Fachmann aus dem Stand der Technik grundsätzlich bekannt.

Bei der Erfindung werden bevorzugt Calciumphosphat-Zemente (hierin auch als CPC bezeichnet), Magnesiumphosphat/Calciumphosphat-Zemente (hierin auch als "Mg-CPC" bezeichnet) oder Magnesiumphosphat-Zemente (hierin auch als "MgPC" bezeichnet) eingesetzt. Die Nomenklatur der mineralischen Zemente beruht auf den bei der Abbindereaktion gebildeten Feststoffen: Calciumphosphat-Zemente bilden bei Aushärtung schwerlösliche Calciumphosphate; Magnesiumphosphat-Zemente bilden bei Aushärtung schwerlösliche Magnesiumphosphate.

Mg-CPC und MgPC enthalten bevorzugt Magnesiumphosphate und/oder Magnesium-Calciumphosphate, insbesondere Mg₃(PO₄)₂ und/oder CaₓMg₃₋ₓ(PO₄)₂ (mit 0 < x < 3). Diese reagieren mit Ammonium- und Phosphationen unter Bildung von Magnesium-Ammonium-Phosphaten. Die dabei ablaufende Reaktion ist in folgender Formel dargestellt:

(5) Mg₃(PO₄)₂ + (NH₄)₂HPO₄ → ₂Mg(NH₄)PO₄ + MgHPO₄

MgPC auf Basis von Magnesiumoxid (MgO) reagiert mit Ammoniumphosphat ebenfalls zu Magnesium-Ammonium-Phosphat. Das im MgPC enthaltene MgO wird vorzugsweise hoch gebrannt, bevorzugt bei Temperaturen > 1500 °C. Mit Ammoniumphosphat erfolgt eine Reaktion gemäß folgender Formel:

(6) MgO + (NH₄)H₂PO₄ → Mg(NH₄)PO₄ + H₂O

In den Reaktionsgleichungen (5) und (6) wurde dabei auf die Nennung von Wasser als Reaktionspartner und als Kristallwasser verzichtet.

Bevorzugte mineralische Knochenzemente enthalten als mineralische Bestandteile Magnesiumphosphate und/oder Magnesium-Calciumphosphate, besonders bevorzugt sind Mg-CPC oder MgPC.

Calciumphosphat-Zemente (CPC) enthalten als Hauptbestandteile mit Wasser reaktive Calciumphosphate, insbesondere α-TCP, β-TCP, tetra-Calciumphosphat, amorphes Calciumphosphat, Mono-Calciumphophat, Calciumhydrogenphosphat und/oder deren Kombinationen. Bei der Abbindereaktion werden schwerlösliche Calciumphosphate gebildet, insbesondere Hydroxylapatit, Calcium-defizientes Hydroxylapatit (CDHA) und/oder Calciumhydrogenphosphat.

Bevorzugt sind CPC, die zu CDHA abbinden und die mit einer wässrigen Ammoniumphosphat-Lösung als Anmischlösung verwendet werden. Besonders bevorzugt sind davon CPC, die als reaktiven Bestandteil α-TCP enthalten.

Weiter bevorzugte mineralische Knochenzemente enthalten Calciumphosphate und/oder Magnesiumphosphate, wobei darin zumindest teilweise Calcium und/oder Magnesium durch zweiwertiges Strontium substituiert sind (strontiumhaltige mineralische Knochenzemente).

Mineralische Knochenzemente enthalten vorzugsweise weitere Bestandteile, die nicht an der Abbindereaktion beteiligt sind. Bevorzugt enthält der mineralische Knochenzement pharmazeutische Wirkstoffe, insbesondere Antibiotika. Alternativ oder in Kombination enthält der mineralische Knochenzement Hilfsstoffe, vorzugsweise solche Hilfsstoffe, die die Viskosität der wässrigen Lösung beeinflussen. Bevorzugte Hilfsstoffe sind wasserlösliche Polymere wie lösliche Stärke und Stärkederivate, Cellulosederivate, Kollagen, Gelatine, PEG, PEG-PPG-Copolymere, wasserlösliche modifizierte Polyacrylate/Polymethacrylate, PVP und/oder PVA. Alternativ oder in Kombination dazu enthält der mineralische Knochenzement partikuläre, vorzugsweise in Wasser schwer lösliche Füllstoffe, insbesondere Stärke, unlösliches Kollagen, unlösliche Gelatine, mineralische Partikel, insbesondere Silikate, und/oder Strontiumsalze.

Die Pulverkomponente des mineralischen Knochenzements liegt in Form eines Pulvers oder in Form einer wasserfreien Paste vor. Bevorzugt ist die Ausführung als wasserfreie Paste. Als wasserfrei wird im Sinne der Erfmdung eine maximale Konzentration von Wasser von 1 Gewichts-%, bevorzugt von maximal 0,1 Gewichts-% (in Bezug auf die Gesamtmasse der Paste) angesehen. In diesem Fall ist das mineralische Knochenzementpulver in einer nicht-wässrigen Trägerflüssigkeit dispergiert.

Nicht-wässrige Trägerflüssigkeiten sind wasserfrei (enthaltend weniger als 1 Gew.-%, vorzugsweise weniger als 0,1 Gew.-% Wasser in Bezug auf die Masse der Trägerflüssigkeit). Als Trägerflüssigkeiten sind wasserlösliche oder nicht-wasserlösliche Flüssigkeiten geeignet.

Bevorzugte Trägerflüssigkeiten sind unpolare oder hydrophobe Stoffe, insbesondere Öle. Bevorzugte Trägerflüssigkeiten sind ausgewählt aus aliphatischen Kohlenwasserstoffen, Estern, Ethern, schwer wasserlöslichen organischen Säuren, schwer wasserlöslichen ein- oder mehrwertigen Alkoholen und deren Kombinationen, wobei die Verbindungen jeweils ein Molekulargewicht von weniger als 2500 g/mol aufweisen. Unter schwer wasserlöslich werden im Sinne der Erfindung Verbindungen verstanden, deren maximale Löslichkeit in Wasser 1,0 mol/l, bevorzugt 0,1 mol/l, beträgt.

Bevorzugt enthält die Paste zudem mindestens einen Abbindebeschleuniger, vorzugsweise Phosphatsalze, organische Säuren (insbesondere Citronensäure, Weinsäure, Itaconsäure, Ascorbinsäure, Milchsäure und/oder Aminosäuren) oder deren Salze. Bevorzugte Abbindebeschleuniger sind Natriumphosphate, Kaliumphosphate, Ammoniumphosphate, Natriumcitrate, Kaliumcitrate, Ammoniumcitrate, Natriumtartrate, Kaliumtartrate und/oder Ammoniumtartrate.

Weiterhin enthält die Paste vorzugsweise mindestens ein Tensid. Bevorzugt sind anionische, kationische, amphotere und/oder nichtionische Tenside.

Die Paste ist vorzugsweise aus dispergierten Feststoffen und Trägerflüssigkeit im Massenverhältnis von mindestens 2 : 1 zusammengesetzt. Bevorzugt ist ein Massenverhältnis von mindestens 3:1. Bevorzugt sind maximal 95 Gew.-% Feststoffe in der Paste enthalten. Im Sinne der Erfindung wird unter der Bezeichnung "Pulverkomponente" auch die Form der wasserfreien Paste verstanden, da diese das mit Wasser reaktive Knochenzementpulver enthält.

Mineralische Knochenzemente können als zweikomponentiges Feststoff /Flüssigkeitssystem (Feststoffkomponente mit mineralischem Knochenzementpulver und Flüssigkomponente enthaltend

Wasser) oder als ein- oder zweikomponentiges Pastensystem vorliegen. Bei zweikomponentigen Pastensystemen ist die Pulverkomponente wie oben beschrieben als wasserfreie Paste formuliert und die wässrige Komponente ist eine wässrige Lösung, reines Wasser oder ebenfalls als Paste formuliert, wobei in der Paste nicht mit Wasser reaktive Feststoffe, insbesondere Füllstoffe, vorzugsweise ausgewählt aus Stärke, wasserunlöslichem Kollagen, wasserunlöslicher Gelatine und/oder mineralischen Partikeln, insbesondere Silikaten, in einer wässrigen Lösung dispergiert sind. Bevorzugt ist die erste Komponente eines zweikomponentigen Pastensystems (Pulverkomponente) wie oben beschrieben als wasserfreie Paste formuliert und die zweite Komponente (wässrige Komponente) ist eine autologe Körperflüssigkeit, insbesondere Knochenmark, eine Suspension autologer Zellen, Blut oder Serum.

Der mineralische Knochenzement, der in der Erfindung eingesetzt wird, enthält wasserlösliche phosphationenhaltige Salze, bevorzugt wasserlösliche ammoniumionen- und phosphationenhaltige Salze. Der mit Wasser angemischte mineralische Knochenzement enthält dann gelöste Phosphationen, vorzugsweise gelöste Ammoniumionen und Phosphationen.

Dies wird dadurch bewerkstelligt, indem
a) wasserlösliche phosphationenhaltige Salze, vorzugsweise wasserlösliche ammoniumionen-und phosphationenhaltige Salze, bevorzugt wasserlösliche Ammoniumphosphate, als Feststoff im Pulver enthalten sind und/oder
b) die wässrige Lösung, mit welcher der Feststoff vermengt wird, Phosphationen, vorzugsweise Ammoniumionen und Phosphationen, bevorzugt gelöste Ammoniumphosphate, enthält und/oder
c) phosphationenhaltige Salze in der Pulverkomponente des mineralischen Knochenzements und/oder in der wässrigen Lösung, mit der der Knochenzement angemischt wird, enthalten sind, wobei vorzugsweise zusätzlich ammoniumionenhaltige Salze in der Pulverkomponente des mineralischen Knochenzements und/oder in der wässrigen Lösung, mit der der Knochenzement angemischt wird, enthalten sind.

Für den Fall b) nachdem dem mineralischen Knochenzement zur Auslösung der Abbindereaktion eine wässrige phosphationenhaltige Lösung zugegeben wird, enthält diese vorzugsweise mindestens 0,1 mol/l, vorzugsweise 0,5 - 5 mol/l, bevorzugt 2 - 5 mol/l Phosphationen. Sind ammonium- und phosphationenhaltige Salze, insbesondere Ammoniumphosphate, in der wässrigen Lösung enthalten, so beträgt die Gesamtkonzentration der ammonium- und phosphationenhaltigen Salze (bzw. Ammoniumphosphate) mindestens 0,1 mol/l, vorzugsweise 0,5 - 5 mol/l, bevorzugt 2 - 5 mol/l. Dadurch wird vorteilhaft erreicht, dass nur ein Teil der Phosphate bei der Abbindereaktion umgewandelt wird und ein restlicher Teil als Depot in der Knochenzementmatrix verbleibt.

In den bevorzugten vorgenannten Fällen a) bis c), in denen Ammoniumionen im mineralischen Knochenzement enthalten sind, enthält der angemischte mineralische Knochenzement Ammoniumionen, vorzugsweise Ammoniumphosphate, in einer Konzentration von vorzugsweise mindestens 0,1 mol/l, bevorzugt 0,5 - 5 mol/l, besonders bevorzugt 2-5 mol/l.

Nach dem erfindungsgemäßen Verfahren werden zunächst magnesiumhaltige metallische Werkstoffe bereitgestellt, deren Oberfläche eine Magnesiumoxidschicht und/oder eine Magnesiumsalzschicht enthält. Unter "Magnesiumsalzschicht" im Sinne der Erfindung ist eine oberflächliche Schicht auf dem magnesiumhaltigen metallischen Werkstoff zu verstehen, welche Magnesiumsalze enthält. Die Magnesiumsalze und/oder Magnesiumoxide, die sich auf der Oberfläche des Werkstoffs befinden, besitzen die Eigenschaft, mit Ammoniumionen und Phosphationen bei Raumtemperatur zu Magnesium-Ammonium-Phosphaten zu reagieren. Da diese Reaktion eine Umwandlung der Magnesiumsalze bzw. Magnesiumoxide in schwerlösliche Magnesium-Ammonium-Phosphate darstellt, wird die Magnesiumoxid- und/oder Magnesiumsalzschicht auch als Konversionsschicht bezeichnet. Bevorzugt sind die Magnesiumsalze Hydroxide, Carbonate oder Hydroxid-Carbonate von Magnesium oder deren Kombinationen untereinander. Besonders bevorzugt weist der magnesiumhaltige metallische Werkstoff auf der Oberfläche MgO, Mg(OH)₂, MgCO₃, Magnesium-Hydroxyd-Carbonat, Mg₃(PO₄)₂, MgHPO₄, MgCl₂, MgSO₄, Magnesiumcitrat oder deren Kombinationen auf.

Die Magnesiumoxidschicht und/oder Magnesiumsalzschicht wird auf dem magnesiumhaltigen metallischen Werkstoff vorzugsweise spontan bei der Lagerung des Werkstoffs gebildet. Alternativ oder in Kombination dazu wird eine Magnesiumsalze und/oder Magnesiumoxid-haltige Schicht durch ein Beschichtungsverfahren auf die Oberfläche des Werkstoffs aufgebracht. Das Beschichten der metallischen Werkstoffe mit Magnesiumoxiden und/oder Magnesiumsalzen ist besonders geeignet, um eine hohe Schichtdicke der Konversionsschicht zu erzielen und damit die Korrosionsbeständigkeit weiter zu verbessern. Die Beschichtung mit den Magnesiumoxiden und/oder Magnesiumsalzen erfolgt dabei bevorzugt elektrochemisch oder wird vorzugsweise durch ein elektrisches Potential unterstützt.

Vorzugsweise wird die Konversionsschicht durch die Behandlung des metallischen magnesiumhaltigen Werkstoffs mit einer organischen oder mineralischen Säure oder der Lösung einer organischen oder mineralischen Säure in Wasser oder einem organischen Lösungsmittel wie Alkohol oder Aceton erzeugt. Alternativ zur Behandlung mit einer Säure erfolgt die Behandlung vorzugsweise mit einem Salz oder einer Salzlösung, wobei das Magnesiumsalz des jeweiligen Anions eine geringere Löslichkeit hat, als das zur Behandlung verwendete Salz eines anderen Kations.

Besonders bevorzugt ist die Behandlung des metallischen magnesiumhaltigen Werkstoffs mit Säuren, deren Magnesiumsalze in der entsprechend gewählten Behandlungsflüssigkeit schwer löslich sind. Vorzugsweise erfolgt die Behandlung des magnesiumhaltigen metallischen Werkstoffs in einer wässrigen oder alkoholischen Zitronensäure-Lösung oder in einer wässrigen oder alkoholischen Lösung eines gut wasserlöslichen Salzes der Zitronensäure, bevorzugt Natriumcitrat. Bei der Behandlung mit Zitronensäure oder Citraten wird auf der Oberfläche des Werkstoffes eine Schicht aus Magnesiumcitrat gebildet. Bei einer anschließenden Kontaktierung mit Ammonium- und Phosphationen wird das Magnesiumcitrat zu Magnesium-Ammonium-Phosphat umgewandelt. Weiter bevorzugt erfolgt die Behandlung des metallischen magnesiumhaltigen Werkstoffs direkt mit Ölsäure oder mit Oleaten, vorzugsweise Natriumoleat in wässriger oder alkoholischer Lösung. Die Konversionsschicht enthält in diesem Fall Magnesiumoleat.

Vorzugsweise wird eine Beschichtung mit Magnesiumsalzen und/oder Magnesiumoxiden auf dem Werkstoff aufgebracht, indem die Magnesiumsalze und/oder Magnesiumoxide in Verbindung mit Hilfsstoffen, vorzugsweise Bindemitteln, auf dem Werkstoff aufgebracht werden. Bei der Beschichtung wird dann eine Schicht auf dem magnesiumhaltigen metallischen Werkstoff ausgebildet, welche Magnesiumsalze und/oder Magnesiumoxide und Hilfsstoffe, vorzugsweise Bindemittel, enthält.

Der magnesiumhaltige metallische Werkstoff mit der Konversionsschicht wird mit phosphationenhaltigem mineralischem Knochenzement kombiniert, wobei die Erzeugung des Verbundmaterials in Gegenwart einer Wasser enthaltenden Zubereitung, vorzugsweise einer wässrigen Lösung, erfolgt, die ammoniumionen- und phosphationenhaltige Salze, vorzugsweise Ammoniumphosphate, enthält.

Durch das Kontaktieren der Magnesiumsalze und/oder Magnesiumoxide auf der Oberfläche des Werkstoffes mit Ammoniumionen und Phosphationen in wässriger Umgebung werden diese in Magnesium-Ammonium-Phosphate umgewandelt, welche eine korrosionshemmende Beschichtung auf der Oberfläche des Werkstoffs bilden. Die korrosionshemmende Beschichtung, die Magnesium-Ammonium-Phosphate enthält, wird auch als Passivierungsschicht bezeichnet. Die gebildeten Magnesium-Ammonium-Phosphate sind schwer wasserlösliche Salze, die Magnesium-, Ammonium-und Phosphationen enthalten. Die durch ein erfindungsgemäßes Verfahren hergestellten, mit Magnesium-Ammonium-Phosphaten beschichteten Werkstoffe weisen auf ihrer Oberfläche eine Schicht auf, vorzugsweise Magnesium-Ammonium-Phosphate der Summenformel Mg(NH₄)PO₄ x 6 H₂O enthält.

Die zur Erzeugung der korrosionshemmenden Beschichtung eingesetzte Wasser enthaltende Zubereitung enthält wasserlösliche Ammoniumionen und Phosphationen in einer Konzentration von jeweils zwischen 0,1 und 5,0 mol/l. Besonders bevorzugt ist der Konzentrationsbereich von 0,5 bis 5,0 mol/l, ganz besonders bevorzugt der Bereich von 2,0 bis 5,0 mol/l.

Das Kontaktieren des magnesiumhaltigen metallischen Werkstoffs mit der ammoniumionen- und phosphationenhaltigen Wasser enthaltenden Zubereitung erfolgt für den Fall, dass dies eine wässrige Lösung ist, bevorzugt durch Einbringen, vorzugsweise Eintauchen, des metallischen Werkstoffs in die wässrige Lösung. Die Kontaktzeit beträgt bevorzugt mindestens 1 min, vorzugsweise mindestens 10 min, besonders bevorzugt mindestens 60 min, ganz besonders bevorzugt zwischen 120 min und 24 h. Vorzugsweise findet das Kontaktieren unter ständigem Rühren der wässrigen Lösung statt.

Werden wasserlösliche ammonium- und phosphationenhaltige Salze als Feststoff in Form einer Beschichtung auf den metallischen Werkstoff aufgebracht, so weisen diese vorzugsweise eine Wasserlöslichkeit von > 10 g/l auf. Durch Zugabe eines wässrigen Lösungsmittels, vorzugsweise Wasser oder mit Wasser angemischtes mineralisches Knochenzementpulver, werden die ammonium- und phosphationenhaltigen Salze gelöst, so dass eine wässrige Zubereitung ausgebildet wird, die Ammonium- und Phosphationen enthält und in Kontakt mit dem metallischen Werkstoff ist.

Wird eine Schicht, die ammonium- und phosphationenhaltige Salze enthält, auf der Oberfläche des magnesiumhaltigen metallischen Werkstoffs vor der Erzeugung des Verbundmaterials aufgebracht, so ist es vorteilhaft, die ammonium- und phosphationenhaltigen Salze in die Konversionsschicht einzubetten. Um dies zu bewerkstelligen, werden Magnesiumsalze und/oder Magnesiumoxide mit ammonium- und phosphationenhaltigen Salzen, vorzugsweise Ammoniumphosphaten, vermischt und als Schicht auf dem magnesiumhaltigen metallischen Werkstoff aufgebracht. Dies geschieht entweder trocken, also durch direktes Aufbringen der vorzugsweise pulverförmigen Feststoffe oder mit Hilfe einer nicht-wässrigen Trägerflüssigkeit. Bevorzugte dafür eingesetzte nicht-wässrige Trägerflüssigkeiten sind Lacke. Als Lacke sind filmbildende, in Wasser schwerlösliche Materialien geeignet, die selbst und deren Abbauprodukte bioverträglich sind. Bevorzugte Lacke sind biodegradierbare Polymere, insbesondere Polymere, die Polylactide, Polyglycolide, Polycaprolactone, Polyhydroxybuttersäure oder Copolymere davon enthalten. Weiter bevorzugte biogegradierbare Polymere sind Polyanhydride. Weiter bevorzugte Lacke sind Fette und Wachse.

Besonders bevorzugt ist die Kombination aus Magnesiumoxiden und Ammoniumphosphaten in Form pulverförmiger Feststoffe, welche feinverteilt in Lacken dispergiert sind. Durch das Aufbringen eines Lacks mit dispergierten Magnesiumsalzen und/oder Magnesiumoxiden und ammonium- und phosphationenhaltigen Salzen wird eine Lackschicht auf dem Werkstoff erzeugt. Bei Zutritt von Wasser gehen die ammonium- und phosphationenhaltigen Salze, vorzugsweise Ammoniumphosphate, in Lösung und reagieren mit den Magnesiumsalzen und/oder Magnesiumoxiden unter Bildung von Magnesium-Ammonium-Phosphaten. Wenn die dabei entstehende Schicht aus Magnesium-Ammonium-Phosphaten verletzt wird oder degradiert, wird bei erneutem Zutritt von Wasser eine neue Schicht ausgebildet.

Alternativ dazu wird eine Schicht die ammonium- und phosphationenhaltige Salze enthält, auf der Oberfläche der Konversionsschicht des magnesiumhaltigen metallischen Werkstoffs vor der Erzeugung des Verbundmaterials aufgebracht, indem ammonium- und phosphationenhaltige Salze, vorzugsweise Ammoniumphosphate, unter Verwendung eines Bindemittels mit anschließender Trocknung aufgebracht werden. Anschließend erfolgt eine Wärmebehandlung bei Temperaturen, welche über dem Schmelzpunkt der aufgebrachten Salze liegen. Vorzugsweise erfolgt die Wärmebehandlung bei einer Temperatur von > 150 °C, vorzugsweise > 190 °C. Dabei schmelzen die aufgebrachten Salze und bilden eine durchgängige Schicht in Form eines glasartigen Überzugs. Bevorzugte Bindemittel sind Phosphate (insbesondere Polyphosphate), wasserlösliche Silikate (insbesondere Wasserglas) und/oder Kieselsole.

Alternativ dazu wird eine Schicht die ammonium- und phosphationenhaltige Salze enthält, auf der Oberfläche der Konversionsschicht des magnesiumhaltigen metallischen Werkstoffs vor der Erzeugung des Verbundmaterials aufgebracht, indem ammonium- und phosphationenhaltige Salze in Kombination mit filmbildenden Polymeren eingesetzt werden. Bevorzugte Polymere sind resorbierbare Polymere. Durch die Beschichtung des Werkstoffs mit Hilfe filmbildender Polymere werden ammonium- und phosphationenhaltige Salze über lange Zeit an der Oberfläche der Beschichtung gehalten.

Bevorzugt wird durch das erfindungsgemäße Verfahren eine Magnesium-Ammonium-Phosphat-Schicht mit einer Schichtdicke von mindestens 0,01 µm auf dem Werkstoff aufgebracht. Besonders bevorzugt ist eine Schichtdicke von 0,01 bis 10 µm.

Werden die Ammoniumphosphate oder ammonium- und phosphationenhaltigen Salze gemeinsam mit Magnesiumsalzen und/oder Magnesiumoxiden in einer nicht-wässrigen Trägerflüssigkeit, insbesondere in einem Lack, aufgebracht, beträgt die Dicke der Schicht vorzugsweise von 20 µm bis 2,0 mm. Diese Schicht enthält sowohl die Magnesiumsalze und/oder Magnesiumoxide als auch die ammonium- und phosphationenhaltigen Salze, vorzugsweise Ammoniumphosphate.

Zur Erzeugung der korrosionshemmenden Beschichtung bei der Herstellung des Knochenimplantats sind die folgenden Varianten bevorzugt, die sowohl allein als auch in beliebiger Kombination untereinander erfolgreich sind:
1. Der magnesiumhaltige metallische Werkstoff, dessen Oberfläche eine Magnesiumoxidschicht und/oder eine Magnesiumsalzschicht aufweist, wird vor der Erzeugung des Verbundmaterials mit einer wässrigen Lösung kontaktiert, die ammoniumionen- und phosphationenhaltige Salze, vorzugsweise Ammoniumphosphate, enthält. Auf diese Weise wird eine korrosionshemmende Beschichtung auf dem metallischen Werkstoff bereits vor dessen Kombination mit dem mineralischen Knochenzement hergestellt.
2. Auf der Oberfläche des magnesiumhaltigen metallischen Werkstoff, dessen Oberfläche eine Magnesiumoxidschicht und/oder eine Magnesiumsalzschicht aufweist, wird zunächst eine Beschichtung aufgebracht, die Ammoniumionen- und Phosphationen-haltige Salze, vorzugsweise Ammoniumphosphate, enthält. Der mit ammoniumionen- und phosphationenhaltigen Salzen beschichtete magnesiumhaltige metallische Werkstoff wird dann vor oder während der Erzeugung des Verbundmaterials mit einer wässrigen Lösung kontaktiert. Durch die Gegenwart des Wassers werden die Ammoniumionen und Phosphationen, die sich auf der Oberfläche des metallischen Werkstoffs befinden, gelöst, so dass *in situ* eine ammoniumionen- und phosphationenhaltige wässrige Lösung entsteht, die zur Bildung der Magnesium-Ammonium-Phosphate auf der Oberfläche des magnesiumhaltigen metallischen Werkstoffs führt. Das wässrige Lösungsmittel ist dabei vorzugsweise eine wässrige Lösung, die vor der Kombination mit dem mineralischen Knochenzement mit dem metallischen Werkstoff in Kontakt gebracht wird oder die wässrige Lösung, mit der die Pulverkomponente des mineralischen Knochenzements kontaktiert (vorzugsweise angemischt) wird. Bei der Beschichtung des magnesiumhaltigen metallischen Werkstoffs mit einer Schicht ammonium- und phosphationenhaltiger Salze ist es somit möglich, die korrosionshemmende Beschichtung vor oder während der Herstellung des Verbundmaterials zu erzeugen.
3. Der magnesiumhaltige metallische Werkstoff, dessen Oberfläche eine Magnesiumoxidschicht und/oder eine Magnesiumsalzschicht aufweist, wird mit einem mineralischen Knochenzement kombiniert, der ammoniumionen- und phosphationenhaltige Salze, vorzugsweise Ammoniumphosphate, enthält. Dabei wird der mineralische Knochenzement (an dieser Stelle ist damit die Pulverkomponente des mineralischen Knochenzements gemeint) vor oder nach der Kombination mit dem magnesiumhaltigen metallischen Werkstoff zur Erzeugung des Verbundmaterials mit einer wässrigen Lösung vermischt. Auf diese Weise wird die korrosionshemmende Beschichtung auf dem magnesiumhaltigen metallischen Werkstoff während der Herstellung des Verbundmaterials erzeugt. Durch die Reaktion des Wassers mit der Pulverkomponente des mineralischen Knochenzements härtet dieser aus, so dass ein festes Verbundmaterial ausgebildet wird.

Wird der magnesiumhaltige metallische Werkstoff mit einem mineralischen Knochenzement kontaktiert, der als wasserfreie Paste ausgeführt ist, so wird der magnesiumhaltige metallische Werkstoff vorzugsweise
- vor der Kombination mit dem mineralischen Knochenzement mit einer korrosionshemmenden Beschichtung mit Magnesium-Ammonium-Phosphaten überzogen und getrocknet, und/oder
- mit mineralischem Knochenzement versetzt, der sowohl ammonium- als auch phosphationenhaltige wasserlösliche Salze enthält (auf diese Weise bildet sich die korrosionshemmende Beschichtung auf dem metallischen Werkstoff bei Zutritt von Wasser aus).

Bevorzugt wird der magnesiumhaltige metallische Werkstoff dessen Oberfläche eine Magnesiumoxidschicht und/oder eine Magnesiumsalzschicht aufweist vor der Erzeugung des Verbundmaterials mit einer wässrigen Lösung kontaktiert, die ammoniumionen- und phosphationenhaltige Salze enthält, so dass zunächst auf dem metallischen Werkstoff eine korrosionshemmende Beschichtung erzeugt wird, die Magnesium-Ammonium-Phosphate enthält. Der so beschichtete magnesiumhaltige metallische Werkstoff wird anschließend mit einem mineralischen Knochenzement kombiniert, der sowohl phosphationen- als auch ammoniumionenhaltige Salze enthält. Dadurch wird vorteilhaft eine besonders hohe Korrosionsbeständigkeit des magnesiumhaltigen metallischen Werkstoffs im Knochenimplantat erzeugt, da auch bei Defekten an der korrosionshemmenden Schicht durch die im Knochenzement enthaltenen Ammonium- und Phosphationen eine besonders effektive Reparatur der Schicht erfolgen kann. Weiterhin sind die mechanischen Eigenschaften des Knochenimplantats erheblich verbessert.

Im erimdungsgemäßen Verfahren ist somit für zwei Reaktionen die Anwesenheit von Wasser erforderlich: für die Erzeugung der korrosionshemmenden Magnesium-Ammonium-Phosphat-Beschichtung auf dem magnesiumhaltigen metallischen Werkstoff und für die Abbindereaktion des mineralischen Knochenzements. Beide Reaktionen können im erfindungsgemäßen Verfahren auch gleichzeitig stattfinden. Die Erzeugung der korrosionshemmenden Beschichtung auf dem Werkstoff kann vor und/oder während der Kombination mit dem mineralischen Knochenzement erfolgen. In jedem Fall werden der magnesiumhaltige metallische Werkstoff und der mineralische Knochenzement so kombiniert, dass die Abbindereaktion des mineralischen Knochenzements noch nicht vor der Kombination abgeschlossen ist.

Die Abbindereaktion des mineralischen Knochenzements wird vorzugsweise durch Zugabe von Wasser oder einer wässrigen Lösung vor der Implantation gestartet. Dies kann auch während des industriellen Herstellprozesses des Knochenimplantats geschehen. In dem Fall, dass der magnesiumhaltige metallische Werkstoff mit einem mineralischen Knochenzement kontaktiert wird, der als wasserfreie Paste ausgeführt ist, ist es möglich, den mit der Knochenzementpaste verbundenen metallischen Werkstoff ohne vorherigen Kontakt mit Wasser oder einer wässrigen Lösung zu implantieren. Die Abbindereaktion des mineralischen Knochenzements wird in diesem Fall spontan im Körper durch das in Körperflüssigkeiten enthaltene Wasser gestartet.

Mit Abschluss der Abbindereaktion ist das feste Verbundmaterial erhältlich, das den mineralischen Knochenzement und den magnesiumhaltigen metallischen Werkstoff enthält. Unter einem Verbundmaterial im Sinne der Erfmdung wird ein fester Körper verstanden, der sowohl das mit Magnesium-Ammonium-Phosphaten beschichtete magnesiumhaltige Metall als auch den mineralischen Knochenzement enthält.

Zur Erzeugung des Verbundmaterials erfolgt die Kombination des magnesiumhaltigen metallischen Werkstoffs mit dem Knochenzement für den Fall, dass zwei Feststoffkomponenten vorliegen (insbesondere Metallspäne und die Pulverkomponente des mineralischen Knochenzements) vorzugsweise durch Vermischen und anschließende Zugabe einer wässrigen Flüssigkeit. Für den Fall, dass der mineralische Knochenzement bereits angemischt wurde und die Abbindereaktion gestartet wurde oder die Pulverkomponente des mineralischen Knochenzements als wasserfreie Paste ausgestaltet ist, erfolgt die Kombination des Werkstoffs mit dem Knochenzement durch in Kontakt bringen der Knochenzement-Paste mit dem metallischen Werkstoff, so dass der mineralische Knochenzement den magnesiumhaltigen metallischen Werkstoff zumindest teilweise umhüllt. Bevorzugt werden der metallische Werkstoff und der mineralische Knochenzement so kombiniert, dass der Werkstoff vollkommen vom Knochenzement umschlossen ist. Im Verbundmaterial liegen der magnesiumhaltige metallische Werkstoff und der mineralische Knochenzement demzufolge so vor, dass zumindest ein Teil des metallischen Werkstoffs von (ausgehärtetem) mineralischem Knochenzement umhüllt ist.

Das erfindungsgemäße Knochenimplantat ist ein festes Verbundmaterial, welches neben dem magnesiumhaltigen metallischen Werkstoff mit der korrosionshemmenden Beschichtung mineralischen Knochenzement enthält, der wasserlösliche phosphationenhaltige Salze enthält. Bevorzugt enthält der mineralische Knochenzement ammoniumionen- und phosphationenhaltige Salze, besonders bevorzugt Ammoniumphosphate.

Die bevorzugten Konzentrationen von wasserlöslichen phosphationenhaltigen Salzen, vorzugsweise wasserlöslichen ammonium- und phosphationenhaltigen Salzen, die als Zusätze im mineralischen Knochenzement eines erfindungsgemäßen Knochenimplantats enthalten sind, betragen insgesamt 0,025 Gewichts-% bis 25 Gewichts-%, vorzugsweise 0,025 Gewichts-% bis 5 Gewichts-%, bezogen auf die Knochenzementmasse. Dies wird bei der Herstellung des erfindungsgemäßen Knochenimplantats vorzugsweise dadurch bewerkstelligt, dass die Pulverkomponente des mineralischen Knochenzements mit einer wässrigen Lösung vermengt wird, die ammoniumionen- und phosphationenhaltige Salze, vorzugsweise Ammoniumphosphate enthält, deren Gesamtkonzentration für CPC bevorzugt 0,1 - 10 Gewichts-% beträgt oder deren Konzentration für MgPC oder Mg-CPC bevorzugt 0,5 bis 5,0 mol/l, weiter bevorzugt 5 bis 60 Gewichts-% beträgt. Das Mischungsverhältnis zwischen Flüssigkeit und Pulver beim Anmischen eines mineralischen Knochenzements beträgt vorzugsweise zwischen 0,3 ml/g und 0,5 ml/g.

Die Dicke der Magnesium-Ammonium-Phosphate enthaltenden Schicht (Passivierungsschicht) auf dem Werkstoff beträgt bevorzugt 2 mm und weniger. Bevorzugt beträgt die Dicke der Passivierungsschicht mehr als 0,01 µm, besonders bevorzugt mehr als 0,1 µm, ganz besonders bevorzugt mindestens 1 µm. Weiter bevorzugt beträgt die Dicke der Passivierungsschicht weniger als 50 µm, vorzugsweise weniger als 20 µm. Weiter bevorzugt beträgt die Dicke der Passivierungsschicht zwischen 0,01 bis 25 µm, weiter bevorzugt zwischen 1 und 10 µm.

Die Passivierungsschicht befindet sich auf der Oberfläche des magnesiumhaltigen metallischen Werkstoffes. Zwischen der oberflächlichen Magnesium-Ammonium-Phosphate enthaltenden Schicht und dem Metall befindet sich bevorzugt eine Magnesiumsalze und/oder Magnesiumoxide enthaltende Schicht (Konversionsschicht). Die Konversionsschicht hat bevorzugt eine Dicke von 0,1 bis 100 µm, vorzugsweise 0,1 bis 50 µm. Dabei sind die Magnesiumsalze und/oder Magnesiumoxide bevorzugt ausgewählt aus MgO, Mg(OH)₂, MgCO₃, Magnesium-Hydroxyd-Carbonat, Mg₃(PO₄)₂, MgHPO₄, MgCl₂, MgSO₄, Magnesiumcitrat und deren Kombinationen. Die Konversionsschicht enthält vorzugsweise außer Magnesiumsalzen und/oder Magnesiumoxiden weiterhin ammoniumionen- und phosphationenhaltige Salze, vorzugsweise Ammoniumphosphate.

Bevorzugte Knochenimplantate enthalten eines der folgendermaßen aufgebauten Verbundmaterialien:
1. Verbundmaterial, welches mineralischen Knochenzement mit phosphationenhaltigen Salzen, vorzugsweise ammonium- und phosphationenhaltigen Salzen (bevorzugt Ammoniumphosphate) und Magnesiumfasern enthält. Knochenimplantate dieser Art werden bei der Anwendung vorzugsweise vor der Implantation in einer an die Knochendefektstelle angepassten Form zugeschnitten.
2. Verbundmaterial, welches mineralischen Knochenzement mit phosphationenhaltigen Salzen, vorzugsweise ammonium- und phosphationenhaltigen Salzen (bevorzugt Ammoniumphosphate) und ein Flächengebilde eines magnesiumhaltigen metallischen Werkstoffs enthält. Bevorzugte Flächengebilde sind Gewebe, Geflechte, Gewirke, Gestricke, Vliese, Vliesstoffe, Membranen, Kordeln, die Drähte des magnesiumhaltigen metallischen Werkstoffs enthalten oder durch Verpressen und/oder Sintern erhältliche Flächengebilde. Das Flächengebilde enthält ein offenporiges Gerüst des magnesiumhaltigen metallischen Werkstoffs und weist ein interkonnektiertes Porensystem auf. Zur Herstellung des Verbundmaterials wird das Flächengebilde mit mineralischem Knochenzement imprägniert, wobei entweder ein mit einer wässrigen Lösung angemischter mineralischer Knochenzement (direkte Herstellung des festen Verbundmaterials) oder mineralischer Knochenzement in Form einer wasserfreien Paste (Knochenzementpulver dispergiert in einer wasserfreien, vorzugsweise hydrophoben, Trägerflüssigkeit wie oben beschrieben) eingesetzt wird. In der zweiten Alternative ist das feste Verbundmaterial durch Kontaktieren mit einer wässrigen Lösung erhältlich.
3. Verbundmaterial, welches mineralischen Knochenzement mit phosphationenhaltigen Salzen, vorzugsweise ammonium- und phosphationenhaltigen Salzen (bevorzugt Ammoniumphosphate) und einen magnesiumhaltigen metallischen Werkstoff in Form einer Platte, eines Nagels, einer Schraube oder eines Cages enthält.
4. Verbundmaterial, welches mineralischen Knochenzement mit phosphationenhaltigen Salzen, vorzugsweise ammonium- und phosphationenhaltigen Salzen (bevorzugt Ammoniumphosphate) und den magnesiumhaltigen metallischen Werkstoff in partikulärer Form (vorzugsweise in Form von Spänen oder Kurzfasern) enthält. Das Verbundmaterial wird bevorzugt hergestellt, indem die Pulverkomponente des mineralischen Knochenzements mit den Spänen oder Kurzfasern vermischt wird und anschließend zur Auslösung der Abbindereaktion mit einer wässrigen Lösung versetzt wird. Alternativ dazu wird das Verbundmaterial vorzugsweise hergestellt, indem ein mineralischer Knochenzement in Form einer wasserfreien Paste (Knochenzementpulver dispergiert in einer wasserfreien, vorzugsweise hydrophoben, Trägerflüssigkeit wie oben beschrieben) mit den Spänen oder Kurzfasern vermischt wird und anschließend zur Auslösung der Abbindereaktion mit einer wässrigen Lösung versetzt wird.

Das erfindungsgemäße Set enthält die erforderlichen Mittel zur Herstellung eines erfindungsgemäßen Knochenimplantats, wobei Wasser oder eine Wasser enthaltende Zubereitung optional enthalten sind. Zumindest sind eine mineralische Knochenzementzubereitung, wasserlösliche phosphationenhaltige Salze und ein magnesiumhaltiger metallischer Werkstoff enthalten. Ist der magnesiumhaltige metallische Werkstoff ohne korrosionshemmende Magnesium-Ammonium-Phosphat-haltige Beschichtung im Set enthalten, so sind auch wasserlösliche ammonium- und phosphationenhaltige Salze Bestandteil des Sets. Ist der im Set enthaltene magnesiumhaltige metallische Werkstoff bereits mit einer Magnesium-Ammonium-Phosphate enthaltenen Beschichtung versehen, so sind optional ammonium- und phosphationenhaltige Salze im Set enthalten.

Von der Bezeichnung Set ist im Sinne der Erfindung sowohl eine ein- oder mehrkomponentige Zubereitung (Implantatmaterial) umfasst, die die oben genannten Bestandteile enthält als auch die verpackten Bestandteile. Bevorzugte Verpackungen, wie Beutel, Spritzen oder dergleichen sind dem Fachmann bekannt. In der Anwendung des erfindungsgemäßen Sets werden die darin enthaltene Knochenzementzubereitung und der magnesiumhaltige metallische Werkstoff mit einem erfindungsgemäßen Verfahren zu einem erfindungsgemäßen Knochenimplantat zusammengefügt.

Für den Fall, dass ein magnesiumhaltiger metallischer Werkstoff mit einer korrosionshemmenden Magnesium-Ammonium-Phosphate enthaltenden Beschichtung enthalten ist, ist ein weiterer Zusatz von ammonium- und phosphationenhaltigen Salzen nicht erforderlich, aber bevorzugt.

Die oben genannten bevorzugten Ausgestaltungen hinsichtlich des magnesiumhaltigen metallischen Werkstoffs, der ammonium- und phosphationenhaltigen Salze und des mineralischen Knochenzements gelten ebenso für das erfindungsgemäße Set.

Das erfindungsgemäße Set ist vorzugsweise als Einkomponentensystem (einkomponentiges Implantatmaterial) ausgestaltet, das die zur Knochenimplantatherstellung erforderlichen Mittel: die Knochenzementzubereitung, den magnesiumhaltigen metallischen Werkstoff und die ammonium- und phosphationenhaltigen Salze in einer einzigen Komponente enthält. Diese Komponente wird zur Herstellung des festen Verbundmaterials in Kontakt mit einer wässrigen Lösung gebracht. Vorzugsweise liegt das einkomponentige Set in Form einer festen pulverfömigen Zubereitung oder in Form einer wasserfreien Paste vor (wobei die wasserfreie Paste gleichermaßen wie oben beschrieben zusammengesetzt ist).

Bevorzugt sind in einem erfindungsgemäßen Set ammonium- und phosphationenhaltige Salze, vorzugsweise Ammoniumphosphate, als Feststoffe in der Knochenzementzubereitung enthalten oder diese liegen in Form einer wässrigen Lösung als separate Komponente des Sets vor. Weiter bevorzugt liegen ammonium- und phosphationenhaltige Salze, vorzugsweise Ammoniumphosphate, als Beschichtung auf dem magnesiumhaltigen metallischen Werkstoff vor.

Bevorzugt ist die Knochenzementzubereitung eines erfindungsgemäßen Sets eine als wasserfreie Paste formulierte Pulverkomponente eines mineralischen Knochenzements (bevorzugt wie oben defmiert). Dabei ist das mineralische Knochenzementpulver in einer nicht-wässrigen, bevorzugt hydrophoben Trägerflüssigkeit dispergiert. Besonders bevorzugt ist in diesem Fall zumindest die Knochenzementzubereitung in eine wasserdichte Verpackung eingebracht.

Weiter bevorzugt liegen der mineralische Knochenzement und der magnesiumhaltige metallische Werkstoff als eine Komponente des Sets vor, die zur Herstellung des Verbundmaterials mit einer wässrigen Lösung kontaktiert wird.

Besonders bevorzugte erfindungsgemäße Sets enthalten eine als wasserfreie Paste formulierte Knochenzementzubereitung und einen partikulären und/oder porösen magnesiumhaltigen metallischen Werkstoff. Wird ein partikulärer metallischer Werkstoff eingesetzt, so ist dieser vorzugsweise in der Knochenzementzubereitung dispergiert. Wird ein poröser metallischer Werkstoff (insbesondere ein offenporiges Flächengebilde wie oben definiert) eingesetzt, so ist die Knochenzementzubereitung bevorzugt in die Poren des metallischen Werkstoffs eingebracht. Auf diese Weise entsteht ein verformbares Verbundmaterial, das erst nach Kontakt mit einer wässrigen Flüssigkeit (insbesondere nach der Implantation im Körper) zur Aushärtung gebracht wird.

Mit der Erfindung ist es möglich, resorbierbare Knochenimplantate, die metallisches Magnesium enthalten, bereitzustellen, die eine verminderte und kontrollierbare Korrosionsgeschwindigkeit aufweisen. Gleichzeitig wird durch den mineralischen Knochenzement eine hohe mechanische Stabilität gewährleistet (insbesondere hohe Druckfestigkeit), so dass die Materialien für den implantiven Einsatz im Knochen besonders geeignet sind.

Anhand folgender Figuren und Ausführungsbeispiele soll die Erfindung näher erläutert werden, ohne die Erfindung auf diese zu beschränken.

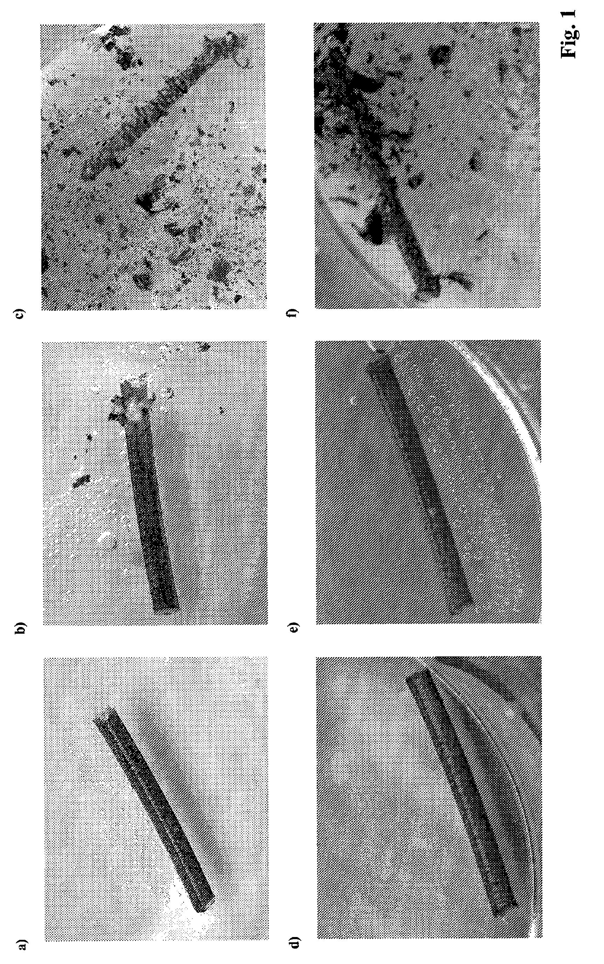

- Fig. 1: Korrosion eines unbehandelten Magnesiumdrahts in simulierter Körperflüssigkeit (Vergleichsbeispiel, a) 0 Stunden, b) 1 Stunde, c) 24 Stunden) und eines mit Ammoniumphosphaten behandelten Magnesiumdrahts (d) 0 Stunden, e) 1 Stunde, f) 24 Stunden).

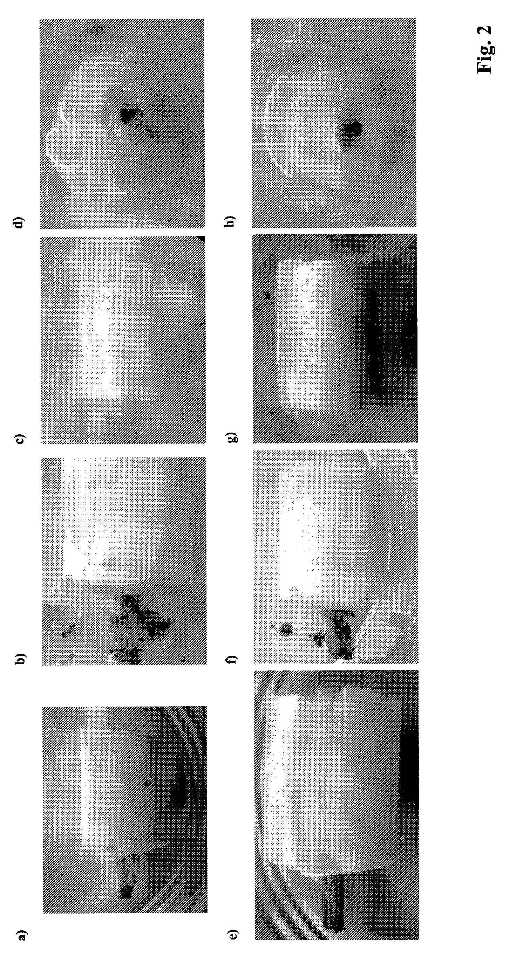

- Fig. 2: Korrosion eines unbehandelten Magnesiumdrahts in Kombination mit Magnesium-Calciumphosphatzement mit Ammoniumphosphat (Vergleichsbeispiel, a) 0 Stunden, b) 24 Stunden, c) und d) (Draufsicht) 6 Wochen) und eines mit Ammoniumphosphaten behandelten Magnesiumdrahts in Kombination mit Magnesium-Calciumphosphatzement mit Ammoniumphosphat (e) 0 Stunden, f) 24 Stunden, g) und h) (Draufsicht) 6 Wochen) in simulierter Körperflüssigkeit.

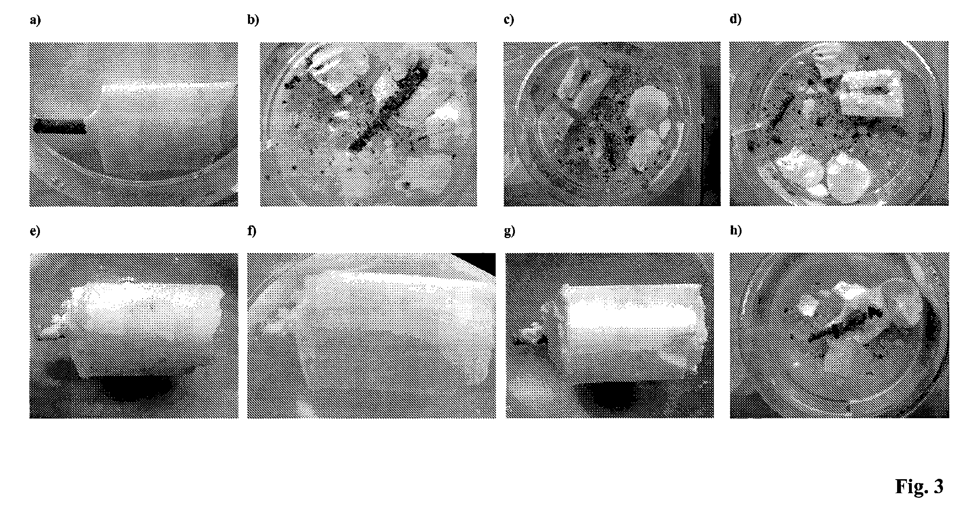

- Fig. 3: Korrosion eines unbehandelten Magnesiumdrahts in Kombination mit Calciumphosphatzement mit Natriumphosphat (Vergleichsbeispiel, a) 0 Stunden, b) 26 Stunden, c) 14 Tage, d) 17 Tage) und eines mit Ammoniumphosphaten behandelten Magnesiumdrahts in Kombination mit Calciumphosphatzement mit Natriumphosphat (e) 0 Stunden, f) 26 Stunden, g) 14 Tage, h) 17 Tage) in simulierter Körperflüssigkeit.

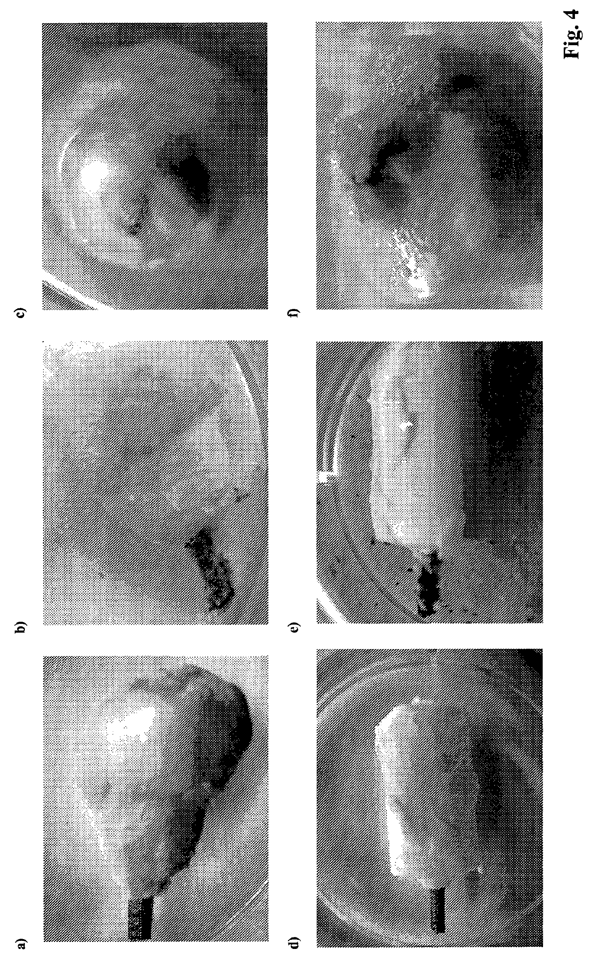

- Fig. 4: Korrosion eines unbehandelten Magnesiumdrahts in Kombination mit Calciumphosphatzement mit Ammoniumphosphat (Vergleichsbeispiel, a) 0 Stunden, b) 28 Stunden, c) 7 Tage) und eines mit Ammoniumphosphaten behandelten Magnesiumdrahts in Kombination mit Calciumphosphatzement mit Ammoniumphosphat (d) 0 Stunden, e) 26 Stunden, f) 7 Tage) in simulierter Körperflüssigkeit.

### Ausführungsbeispiel 1: Beschichtung von Magnesium-Draht mit Magnesium-Ammonium-Phosphaten

Magnesiumdraht (Mg-Draht) mit einer Reinheit von 99,9 % und einem Durchmesser von 1,6 mm (Fa: Drahtwerk ELISENTAL W. Erdmann GmbH & Co., Neuenrade) wurde bei Raumtemperatur gelagert und anschließend in einer 2,0 mol/l di-Ammoniumhydrogenphosphatlösung 2 h unter ständigem Rühren inkubiert. Der so behandelte Mg-Draht wurde in simulierter Körperflüssigkeit (SBF) bei 37 °C für 24 h inkubiert. Als Vergleichsprobe diente ein unbehandelter Magnesium-Draht.

Die Korrosion des nach einem erfindungsgemäßen Verfahren beschichteten Magnesiumdrahts im Vergleich zum unbehandelten Draht ist in Figur 1 dargestellt. Während der unbehandelte Mg-Draht unmittelbar korrodiert (Blasenbildung), kann bei dem erfindungsgemäß behandelten Magnesiumdraht eine Verzögerung der Korrosion festgestellt werden. Dennoch ist bereits nach 24 h eine deutliche Korrosion auch bei dem behandelten Mg-Draht festzustellen. Dies zeigt, dass die Beschichtung mit Magnesium-Ammonium-Phosphaten allein in Abwesenheit von mineralischem Knochenzement noch keine ausreichende Korrosionsbeständigkeit liefern kann.

### Ausführungsbeispiel 2: Implantatmaterial, enthaltend einen mit Magnesium-Ammonium-Phosphaten beschichteten Magnesium-Draht und Magnesium-Calciumphosphat-Zement mit Ammoniumphosphaten

Magnesiumdraht (Mg-Draht) mit einer Reinheit von 99,9 % und einem Durchmesser von 1,6 mm (Fa. Drahtwerk ELISENTAL W. Erdmann GmbH & Co., Neuenrade) wurde bei Raumtemperatur gelagert und anschließend in einer 2,0 mol/l di-Ammoniumhydrogenphosphatlösung 2 h unter ständigem Rühren inkubiert.

Magnesium-Calciumphosphat-Zementpulver wurde mit 3,5 mol/l Ammoniumphosphat-Lösung (2,0 mol/l (NH₄)₂HPO₄ + 1,5 mol/L (NH₄)H₂PO₄) im Verhältnis Flüssigkeit/Pulver = 0,4 ml/g vermischt und mit unbehandelten Mg-Draht bzw. mit Ammoniumphosphat behandelten Mg-Draht (Vergleichsbeispiel) kombiniert. An einem Ende des hergestellten zylindrischen Formkörpers (Ø=10 mm, H = 20 mm) ragte ein ca. 2 mm langes Stück des unbehandelten bzw. behandelten Mg-Drahts heraus.

Die hergestellten Draht-Zement-Komposite wurden in simulierter Körperflüssigkeit (SBF) bei 37 °C inkubiert. Die SBF wurde mit TRIS gepuffert und 2x pro Woche gewechselt.

Das Korrosionsverhalten beider Materialien ist in Fig. 2 dargestellt.

Auch nach 6 Wochen bleibt sowohl das Komposit aus unbehandeltem Mg-Draht und Magnesium-Calciumphosphat-Zement als auch das Komposit aus behandeltem Mg-Draht und Magnesium-Calciumphosphat-Zement als solches erhalten. Das freie Drahtende des unbehandelten Magnesiumdrahts korrodierte schneller, als das freie Drahtende des beschichteten Magnesiumdrahts.

Überraschend wurde festgestellt, dass zwar das freiliegende Ende des Magnesiumdrahts korrodierte, die Korrosion des Magnesiums sich jedoch nicht wesentlich innerhalb des mineralischen Knochenzements fortsetzte. Die Korrosion des Magnesiumdrahtes wird durch die Passivierungsschicht aus Magnesium-Ammonium-Phosphaten, welche sich an der Grenze vom Magnesium zum Knochenzement befindet, gehemmt.

Während der Inkubation lagerten sich sowohl am Zementkörper als auch an abgebrochenen Mg-DrahtStücken weiße Salzkristalle aus Magnesium-Ammonium-Phosphat ab. Dies zeigt deutlich, dass der mineralische Knochenzement, der Ammoniumphosphate enthält, zur Verringerung der Korrosionsgeschwindigkeit beiträgt.

### Ausführungsbeispiel 3: Implantatmaterial, enthaltend einen mit Magnesium-Ammonium-Phosphaten beschichteten Magnesium-Draht und Calciumphosphat-Zement mit Natriumphosphat

Magnesiumdraht (Mg-Draht) mit einer Reinheit von 99,9 % und einem Durchmesser von 1,6 mm (Fa. Drahtwerk ELISENTAL W. Erdmann GmbH & Co., Neuenrade) wurde bei Raumtemperatur gelagert und anschließend in einer 2,0 mol/l di-Ammoniumhydrogenphosphatlösung 2 h unter ständigem Rühren inkubiert.

Calciumphosphat Zementpulver wurden mit 4 % (w/v) di-Natriumhydrogenphosphat-Lösung im Verhältnis Flüssigkeit/Pulver = 0,4 ml/g vermischt und dafür genutzt, sowohl unbehandelten Mg-Draht als auch mit Ammoniumphosphat behandelten Mg-Draht zu umhüllen. An einem Ende des hergestellten zylindrischen Formkörpers (Ø=10 mm, H = 20 mm) ragte ein ca. 2 mm langes Stück des unbehandelten bzw. behandelten Mg-Drahts heraus.

Die hergestellten Draht-Zement-Komposite wurden in simulierter Körperflüssigkeit (SBF) bei 37 °C inkubiert. Die SBF wurde mit TRIS gepuffert und 2x pro Woche gewechselt.

Das Korrosionsverhalten beider Materialien ist in Fig. 3 dargestellt.

Bereits nach 26 h wurde das Komposit aus unbehandeltem Mg-Draht und mit di-Natriumhydrogenphosphat-Lösung angemischtem Calciumphosphat Zement durch die während der Korrosion des Mg-Drahtes gebildeten Korrosionsprodukte gesprengt. In Abwesenheit von Ammoniumionen kann somit keine ausreichende Korrosionsbeständigkeit des Magnesiumdrahts erreicht werden, um eine Eignung für Knochenimplantate zu ermöglichen.

Nach 17 Tagen wurde das Komposit aus behandeltem Mg-Draht und mit di-Natriumhydrogenphosphat-Lösung angemischtem Calciumphosphat Zement durch die während der Korrosion des Mg-Drahtes gebildeten Korrosionsprodukte gesprengt. Durch die vorherige Behandlung des Magnesiumdrahts in wässriger Lösung mit Ammoniumphosphaten konnte die Korrosionsbeständigkeit somit signifikant erhöht werden, so dass das Komposit dadurch mehrere Wochen stabil bleibt.

Die dargestellten Ergebnisse zeigen somit, dass erst durch die Gegenwart von ammoniumionen- und phosphationenhaltigen Salzen bei der Herstellung des Verbundmaterials ein signifikanter Korrosionsschutz festgestellt wird, der eine Haltbarkeit des magnesiumhaltigen Implantats von mehreren Wochen ermöglicht.

### Ausführungsbeispiel 4: Implantatmaterial, enthaltend einen mit Magnesium-Ammonium-Phosphaten beschichteten Magnesium-Draht und Calciumphosphat-Zement mit Ammoniumphosphaten

Magnesiumdraht (Mg-Draht) mit einer Reinheit von 99,9 % und einem Durchmesser von 1,6 mm (Fa. Drahtwerk ELISENTAL W. Erdmann GmbH & Co., Neuenrade) wurde bei Raumtemperatur gelagert und anschließend in einer 2,0 mol/l di-Ammoniumhydrogenphosphatlösung 2 h unter ständigem Rühren inkubiert.

Calciumphosphat-Zementpulver wurden mit 3,5 mol/l di-Ammoniumhydrogenphosphatlösung im Verhältnis Flüssigkeit/Feststoff = 0,4 ml/g vermischt und mit unbehandelten Mg-Draht (Vergleichsbeispiel) bzw. mit Ammoniumphosphat behandelten Mg-Draht kombiniert. An einem Ende des hergestellten zylindrischen Formkörpers (Ø=10 mm, H = 20 mm) ragte ein ca. 2 mm langes Stück des unbehandelten bzw. behandelten Mg-Drahts heraus.

Die hergestellten Draht-Zement-Komposite wurden in simulierter Körperflüssigkeit (SBF) bei 37 °C inkubiert. Die SBF wurde mit TRIS gepuffert und 2x pro Woche gewechselt.

Das Korrosionsverhalten beider Materialien ist in Fig. 4 dargestellt.

Das freie Drahtende des unbehandelten Magnesiumdrahts korrodierte bereits nach 28 Stunden. Das freie Drahtende des beschichteten Magnesiumdrahts begann erst deutlich später mit der Korrosion.

Sowohl das mit Ammoniumphosphat behandelte als auch das unbehandelte freiliegende Magnesiumdrahtende zeigen innerhalb einer Woche deutlich Ablagerungen von Magnesium-Ammonium-Phosphat-Salzkristallen, die eine rasche Korrosion des Magnesiumdrahtes verhindern. Die dem Zement beigemischten überschüssigen Ammoniumphosphate gehen teilweise durch die Lagerung in SBF in Lösung und stehen somit einer Beschichtung am freien Drahtende zur Verfügung. Die Salzablagerungen aus Magnesium-Ammonium-Phosphaten sind besonders gut in den Fig. 4 c) und f) zu erkennen. Dies zeigt deutlich, dass der mineralische Knochenzement, der Ammoniumphosphate enthält, zur Verringerung der Korrosionsgeschwindigkeit beiträgt.

### Ausführungsbeispiel 5: Verbundmaterial aus Calciumphosphat-Zement und mit Ammoniumphosphaten behandelten Magnesiumspänen

Magnesiumspäne (Mg-Späne) einer Größe von 0,06 - 0,3 mm (Merck, Darmstadt) wurden bei Raumtemperatur gelagert und anschließend in einer 2,0 mol/l di-Ammoniumhydrogenphosphatlösung 2 h unter ständigem Rühren inkubiert.

Die so behandelten Mg-Späne wurden anschließend abgetrennt, getrocknet und in 10, 20 und 30 Masse-% zu Calciumphosphat-Zementpulver zugesetzt und homogen vermischt.

Aus den daraus resultierenden Metall-Zement-Pulvermischungen wurden mit 3,5 mol/l di-Ammoniumhydrogenphosphat-Lösung in einem Verhältnis von 0,4 ml Lösung pro 1 g Zementpulver Verbundmaterialien (Quader, 6 x 6 x 12 mm) hergestellt.

Die hergestellten Verbundmaterialien wurden in simulierter Körperflüssigkeit (SBF) bei 37 °C inkubiert. Die SBF wurde mit TRIS gepuffert und 2x pro Woche gewechselt.

Die Druckfestigkeit der Verbundmaterialien wurde an einer Universalprüfmaschine (INSTRON) bestimmt und ist in Tabelle 1 zusammengefasst. Dabei zeigen (A) Calciumphosphat-Zement, (B) Calciumphosphat-Zement mit 10 Gewichts-% Magnesiumspänen, (C) Calciumphosphat-Zement mit 20 Gewichts-% Magnesiumspänen und (D) Calciumphosphat-Zement mit 30 Gewichts-% Magnesiumspänen.

**Tabelle 1: Druckfestigkeiten [MPa] von CPC-Magnesium-Formkörpern**

| | Druckfestigkeiten [MPa] | | | |
|---|---|---|---|---|
| Zeit | (A) | (B) | (C) | (D) |
| 0 h | 24,26 +/- 5,10 | 25,12 +/- 6,72 | 21,98 +/- 6,03 | 30,29 +/- 3,23 |
| 2 Wochen | 21,23 +/- 3,34 | 30,59 +/- 7,55 | 26,76 +/- 5,59 | 36,03 +/- 9,41 |

Es ist nach 2 Wochen ein leichter Festigkeitsanstieg der Formkörper zu verzeichnen, der vermutlich auf die Bildung von festem Magnesium-Ammonium-Phosphat zurückzuführen ist.

### Ausführungsbeispiel 6: Verbundmaterial aus Magnesium-Calciumphosphat-Zement und mit Ammoniumphosphat behandelten Magnesiumspänen

Magnesiumspäne (Mg-Späne) einer Größe von 0,06 - 0,3 mm, (Merck, Darmstadt) wurden bei Raumtemperatur gelagert und anschließend in einer 2,0 mol/l di-Ammoniumhydrogenphosphatlösung 2 h unter ständigem Rühren inkubiert.

Die so behandelten Mg-Späne wurden anschließend abgetrennt, getrocknet und in 10, 20 und 30 Masse-% Magnesium-Calciumphosphat-Zementpulver zugesetzt und homogen vermischt.

Aus den daraus resultierenden Metall-Zement-Pulvermischungen wurden mit 3,5 mol/l Ammoniumphosphat-Lösung (2 mol/L (NH₄)₂HPO₄ + 1,5 mol/L (NH₄)H₂PO₄) in einem Verhältnis von 0,4 mL Lösung pro 1 g Zementpulver Verbundmaterialien (Quader, 6 x 6 x 12 mm) hergestellt.

Die hergestellten Verbundmaterialien wurden in simulierter Körperflüssigkeit (SBF) bei 37 °C inkubiert. Die SBF wurde mit TRIS gepuffert und 2x pro Woche gewechselt.

Die Druckfestigkeit der Verbundmaterialien wurde an einer Universalprüfmaschine (INSTRON) bestimmt und ist in Tabelle 2 zusammengefasst. Dabei zeigen (A) Magnesium-Calciumphosphat-Zement, (B) Magnesium-Calciumphosphat-Zement mit 10 Gewichts-% Magnesiumspänen, (C) Magnesium-Calciumphosphat-Zement mit 20 Gewichts-% Magnesiumspänen und (D) Magnesium-Calciumphosphat-Zement mit 30 Gewichts-% Magnesiumspänen.

Die mit Ammoniumphosphat-Lösung hergestellten Formkörper mit Magnesium-Calciumphosphat Zementpulver blieben für mehrere Wochen formstabil.

Des Weiteren ist nach 2 Wochen sogar ein leichter Festigkeitsanstieg zu verzeichnen, der vermutlich auf die Bildung von festem Magnesium-Ammonium-Phosphat in dem Formkörper zurückzuführen ist.

**Tabelle 2: Druckfestigkeiten [MPa] von MgCPC-Magnesium-Formkörpern**

| | Druckfestigkeiten [MPa] | | | |
|---|---|---|---|---|
| Zeit | (A) | (B) | (C) | (D) |
| 0 h | 69,99 +/- 6,83 | 79,33 +/- 13,23 | 82, 53 +/- 3,91 | 82,88 +/- 15,05 |
| 2 Wochen | 64,40 +/- 11,24 | 85,84 +/- 7,96 | 86,60 +/- 6,01 | 98,78 +/- 14,68 |

### Ausführungsbeispiel 7: Verbundmaterial aus Calciumphosphat-Zement und unbehandelten Magnesiumspänen

Magnesiumspäne einer Größe von 0,06 - 0,3 mm (Merck, Darmstadt) wurden bei Raumtemperatur gelagert. Die Magnesiumspäne wurden in Calciumphosphat-Zementpulver homogen eingemischt, so dass der Anteil der Magnesiumspäne an der Mischung 30 Gew.-% betrug.

Aus den daraus resultierenden Metall-Zement-Pulvermischungen wurden mit 3,5 mol/l di-Ammoniumhydrogenphosphat-Lösung in einem Verhältnis von 0,625 ml Lösung pro 1 g Zementpulver Verbundmaterialien (Quader, 6 x 6 x 12 mm) hergestellt.

Die hergestellten Verbundmaterialien wurden in simulierter Körperflüssigkeit (SBF) bei 37 °C inkubiert. Nach einer Woche in SBF waren die hergestellten Verbundmaterialien stabil und es war keine Gasblasenbildung zu sehen.

### Ausführungsbeispiel 8: Verbundmaterial aus Magnesium-Calciumphosphat-Zement und unbehandelten Magnesiumspänen

Magnesiumspäne einer Größe von 0,06 - 0,3 mm (Merck, Darmstadt) wurden bei Raumtemperatur gelagert. Die Magnesiumspäne wurden in Magnesium-Calciumphosphat-Zementpulver homogen eingemischt, so dass der Anteil der Magnesiumspäne an der Mischung 30 Gew.-% betrug.

Aus den daraus resultierenden Metall-Zement-Pulvermischungen wurden mit 3,5 mol/l di-Ammoniumhydrogenphosphat-Lösung in einem Verhältnis von 0,625 ml Lösung pro 1 g Zementpulver Verbundmaterialien (Quader, 6 x 6 x 12 mm) hergestellt.

Die hergestellten Verbundmaterialien wurden in simulierter Körperflüssigkeit (SBF) bei 37 °C inkubiert. Nach einer Woche in SBF waren die hergestellten Verbundmaterialien ohne Gasbildung stabil.

### Ausführungsbeispiel 9: Verbundmaterial aus pastösem Calciumphosphatzement und mit Ammoniumphosphaten behandelten Magnesiumspänen

Magnesiumspäne einer Größe von 0,06 - 0,3 mm (Merck, Darmstadt) wurden bei Raumtemperatur gelagert und anschließend in einer 2,0 mol/l di-Ammoniumhydrogenphosphatlösung 2 h unter ständigem Rühren inkubiert. Die so behandelten Magnesiumspäne wurden von der Lösung abgetrennt und getrocknet.

Die behandelten Späne wurden in einen pastösen Calciumphosphat-Zement (80 Gew.-% Calciumphosphat-Zementpulver in einem mittelkettigen Triglycerid (wasserfreie Trägerflüssigkeit) bezogen auf die Gesamtmasse der Paste, enthaltend Kaliumhydrogenphosphat, Cetylphosphat und ethoxyliertes Ricinusöl homogen eingemischt. Der Massegehalt der Magnesiumspäne an der so hergestellten Metall-Zementpaste betrug 10 Gew.-% (in Bezug auf die Gesamtmasse).

Die so hergestellte Metall-Zementpaste wurde mit einer wässrigen Lösung versetzt, wodurch die Abbindereaktion des Calciumphosphatzements ausgelöst wird. Es wurden Verbundmaterialien (Quader, 6 x 6 x 12 mm) hergestellt. Hierbei wurde 0,8 g Metall-Zementpaste mit 500 µL SBF vermischt. Nach 60 Minuten initialer Aushärtung wurden die hergestellten Verbundmaterialien in simulierter Körperflüssigkeit (SBF) bei 37 °C inkubiert. Nach einer Woche in SBF waren die hergestellten Verbundmaterialien ohne Gasbildung stabil.

### Ausführungsbeispiel 10: Verbundmaterial aus pastösem Calciumphosphatzement und unbehandelten Magnesiumspänen

Magnesiumspäne einer Größe von 0,06 - 0,3 mm (Merck, Darmstadt) wurden bei Raumtemperatur gelagert. Die Magnesiumspäne wurden in einen pastösen Calciumphosphat-Zement (80 Gew.-% Calciumphosphat-Zementpulver in einem mittelkettigen Triglycerid (wasserfreie Trägerflüssigkeit) bezogen auf die Gesamtmasse der Paste, enthaltend di-Ammoniumhydrogenphosphat, Cetylphosphat und ethoxyliertes Ricinusöl homogen eingemischt. Der Massegehalt der Magnesiumspäne an der so hergestellten Metall-Zementpaste betrug 10 Gew.-% (in Bezug auf die Gesamtmasse).

Die so hergestellte Metall-Zementpaste wurde mit einer wässrigen Lösung versetzt, wodurch die Abbindereaktion des Calciumphosphatzements ausgelöst wird. Es wurden Verbundmaterialien (Quader, 6 x 6 x 12 mm) hergestellt. Hierbei wurde 0,8 g Metall-Zementpaste mit 500 µL SBF vermischt. Nach 60 Minuten initialer Aushärtung wurden die hergestellten Verbundmaterialien in simulierter Körperflüssigkeit (SBF) bei 37 °C inkubiert. Nach einer Woche in SBF waren die hergestellten Verbundmaterialien ohne Gasbildung stabil.

## Patentansprüche

1. Verfahren zur Herstellung eines Knochenimplantats, welches einen magnesiumhaltigen metallischen Werkstoff mit einer Magnesium-Ammonium-Phosphate enthaltenden korrosionshemmenden Beschichtung und mineralischen Knochenzement umfasst, wobei ein magnesiumhaltiger metallischer Werkstoff, dessen Oberfläche eine Magnesiumoxidschicht und/oder eine Magnesiumsalzschicht aufweist, zur Erzeugung eines festen Verbundmaterials, welches den mineralischen Knochenzement und den magnesiumhaltigen metallischen Werkstoff enthält, mit mineralischem Knochenzement, der mineralische Pulverbestandteile, die in Gegenwart von Wasser zu einem Feststoff abbinden und wasserlösliche phosphationenhaltige Salze enthält, kombiniert wird, wobei der magnesiumhaltige metallische Werkstoff vor und/oder während der Kombination mit dem mineralischen Knochenzement in Gegenwart wasserlöslicher ammoniumionen- und phosphationenhaltiger Salze mit Wasser in Kontakt gebracht wird und der mineralische Knochenzement zur Abbindung mit Wasser in Kontakt gebracht wird.

2. Verfahren nach Anspruch 1, wobei der magnesiumhaltige metallische Werkstoff vor der Erzeugung des Verbundmaterials mit einer wässrigen Lösung kontaktiert wird, die ammoniumionen- und phosphationenhaltige Salze enthält.

3. Verfahren nach einem der vorhergehenden Ansprüche, wobei auf der Oberfläche des magnesiumhaltigen metallischen Werkstoffs vor der Erzeugung des Verbundmaterials eine Schicht die ammoniumionen- und phosphationenhaltige Salze enthält, aufgebracht wird und der mit ammoniumionen- und phosphationenhaltigen Salzen beschichtete magnesiumhaltige metallische Werkstoff vor oder während der Erzeugung des Verbundmaterials mit einer wässrigen Lösung kontaktiert wird.

4. Verfahren nach einem der vorhergehenden Ansprüche, wobei der mineralische Knochenzement in Form einer wasserfreien Paste vorliegt, die mineralisches Knochenzementpulver enthält, welches in einer wasserfreien Trägerflüssigkeit dispergiert ist.

5. Verfahren nach einem der vorhergehenden Ansprüche, wobei die ammoniumionen- und phosphationenhaltige Salze Ammoniumphosphate sind, bevorzugt ausgewählt aus (NH₄)₃PO₄, (NH₄)₂HPO₄, (NH₄)H₂PO₄, Ammoniumsalzen der di-, oligo- und polymeren Phosphorsäure, sowie der teilweise substituierten Phosphorsäure, insbesondere Ammonium-Glycerophosphat, und deren Mischungen untereinander.

6. Knochenimplantat umfassend einen magnesiumhaltigen metallischen Werkstoff und mineralischen Knochenzement, der wasserlösliche phosphationenhaltige Salze enthält, wobei der magnesiumhaltige metallische Werkstoff eine Beschichtung aufweist, die Magnesium-Ammonium-Phosphate enthält.

7. Knochenimplantat nach Anspruch 6, **dadurch gekennzeichnet, dass** die Magnesium-Ammonium-Phosphate enthaltende Schicht eine Dicke von mehr als 0,01 µm und weniger als 50 µm aufweist.

8. Knochenimplantat nach Anspruch 6 oder 7, **dadurch gekennzeichnet, dass** sich zwischen dem magnesiumhaltigen metallischen Werkstoff und der Schicht aus Magnesium-Ammonium-Phosphaten eine Schicht befindet, welche Magnesiumoxid und/oder Magnesiumsalze enthält.

9. Knochenimplantat nach einem der Ansprüche 6 bis 8, wobei der magnesiumhaltige metallische Werkstoff in Form von
- Fasern, Fäden, Filamenten, Drähten,
- zu Geweben, Geflechten, Gewirken, Gestricken, Vliesen, Vliesstoffen, Membranen oder Kordeln verarbeiteten Drähten oder Fäden,
- aus Fasern, Fäden, Filamenten oder Drähten erhältlichen Flächengebilden,
- perforierten Blechen oder Streckmetall oder aus perforierten Blechen oder Streckmetall hergestellten Formkörpern oder
- einem offenzelligen Metallschaum vorliegt.

10. Set zur Herstellung eines Knochenimplantats, enthaltend
a) eine Knochenzementzubereitung, umfassend mineralisches Knochenzementpulver und wasserlösliche phosphationenhaltige Salze, und
b) einen magnesiumhaltigen metallischen Werkstoff mit einer Magnesiumoxidschicht und/oder einer Magnesiumsalzschicht und ammonium- und phosphationenhaltige Salze, vorzugsweise Ammoniumphosphate, oder
c) einen magnesiumhaltigen metallischen Werkstoff mit einer Magnesium-Ammonium-Phosphate enthaltenden Beschichtung und ggf. ammonium- und phosphationenhaltige Salze.

11. Set nach Anspruch 10, wobei die Knochenzementzubereitung in Form einer wasserfreien Paste vorliegt, die mineralisches Knochenzementpulver dispergiert in einer wasserfreien Trägerflüssigkeit enthält.

12. Set nach Anspruch 10 oder 11, wobei ammonium- und phosphationenhaltige Salze, vorzugsweise Ammoniumphosphate, als Feststoffe enthalten sind oder in Form einer wässrigen Lösung als von der Knochenzementzubereitung separat verpackter Bestandteil des Sets vorliegen.

13. Set nach Anspruch 10 oder 11, wobei ammonium- und phosphationenhaltige Salze, vorzugsweise Ammoniumphosphate, als Beschichtung auf dem magnesiumhaltigen metallischen Werkstoff vorliegen.

14. Set nach einem der Ansprüche 10 bis 13, wobei der mineralische Knochenzement und der magnesiumhaltige metallische Werkstoff als eine Komponente des Sets vorliegen, die zur Herstellung des Verbundmaterials mit einer wässrigen Lösung kontaktiert wird.

15. Set nach einem der Ansprüche 10 bis 14, wobei der mineralische Knochenzement als Zweikomponenten-System ausgestaltet ist, das eine wasserfreie Komponente mit mineralischem Knochenzementpulver und eine Wasser enthaltende Komponente enthält.

## Claims

1. A method for producing a bone implant which comprises a magnesium-containing metallic material with a corrosion-inhibiting coating that contains magnesium ammonium phosphates and comprises mineral bone cement, wherein, for producing a solid composite material containing the mineral bone cement and the magnesium-containing metallic material, magnesium-containing metallic material, whose surface comprises a magnesium oxide layer and/or a magnesium salt layer, is combined with mineral bone cement that contains mineral powder components, which set in the presence of water to a solid, and contains water-soluble phosphate ion containing salts, wherein the magnesium-containing metallic material is contacted with water in the presence of water-soluble ammonium ion containing and phosphate ion containing salts before and/or during combining with the mineral bone cement and the mineral bone cement is contacted with water for setting.

2. A method according to claim 1, wherein the magnesium-containing metallic material is contacted prior to producing the composite material with an aqueous solution which contains ammonium ion containing and phosphate ion containing salts.

3. A method according to one of the preceding claims, further comprising the step of applying on the surface of the magnesium-containing metallic material, prior to producing the solid composite material, a layer containing ammonium ion containing and phosphate on containing salts and contacting the magnesium-containing metallic material coated with ammonium ion containing and phosphate ion containing salts with an aqueous solution prior to or during producing the solid composite material.

4. A method according to one of the preceding claims, wherein the mineral bone cement is present in the form of an anhydrous paste which contains mineral bone cement powder dispersed in an anhydrous carrier liquid.

5. A bone implant according to one of the preceding claims, wherein the ammonium ion containing and the phosphate ion containing salts are ammonium phosphates, preferably selected from (NH₄)₃PO₄, (NH₄)₂HPO₄ (NH₄)H₂PO₄ ammonium salts of the dimeric, oligomeric and polymeric phosphoric acid and of partially substituted phosphoric acid, in particular ammonium glycerophosphate, and mixtures with each other.

6. A bone implant comprising a magnesium-containing metallic material and a mineral bone cement which contains water-soluble phosphate ion containing salts, wherein the magnesium-containing metallic material comprises a coating which contains magnesium ammonium phosphates.

7. A bone implant according to claim 6, wherein the layer containing ammonium phosphates has a thickness of more than 0.01 µm and less than 50 µm.

8. A bone implant according to claim 6 or 7, wherein, between the magnesium-containing metallic material and the layer of magnesium ammonium phosphates, a layer is provided which contains magnesium oxide and/or magnesium salts.

9. A bone implant according to one of the claims 6 to 8, wherein the magnesium-containing metallic material is present in the form of
- fibers, threads, filaments, wires,
- wires or threads processed to woven fabrics, braided fabrics, knit fabrics, knitted materials, fleeces, nonwovens, membranes or cords
- web materials obtainable from fibers, threads, filaments or wires,
- perforated sheet metal or expanded metal or shaped bodies that are formed from perforated sheet metal or expanded metal, or
- an open-cell metal foam.

10. A kit for producing a bone implant, comprising
a) a bone cement preparation comprising mineral bone cement powder and water-soluble phosphate ion containing salts, and
b) a magnesium-containing metallic material with a magnesium oxide layer and/or a magnesium salt layer and ammonium ion containing and phosphate ion containing salts, preferably ammonium phosphates, or
c) a magnesium-containing metallic material with a coating containing magnesium ammonium phosphates and optionally ammonium ion containing and phosphate ion containing salts.

11. A kit according to claim 10, wherein the bone cement preparation is present in the form of an anhydrous paste which contains mineral bone cement powder dispersed in an anhydrous carrier liquid.

12. A kit according to claim 10 or 11, wherein ammonium ion containing and phosphate ion containing salts, preferably ammonium phosphates, are contained as solids or are present in the form of an aqueous solution as a component that is packaged separate from the bone cement preparation of the kit.

13. A kit according to claim 10 or 11, wherein the ammonium ion containing and phosphate ion containing salts, preferably ammonium phosphates, are present as a coating on the magnesium-containing metallic material.

14. A kit according to one of the claims 10 to 13, wherein the mineral bone cement and the magnesium-containing metallic material are present as a component of the kit that is contacted with an aqueous solution for producing a solid composite material.

15. A kit according to one of the claims 10 to 14, wherein the mineral bone cement is configured as a two-component system which contains an anhydrous component with mineral bone cement powder and a water-containing component.

## Revendications

1. Procédé de fabrication d'un implant osseux qui comprend un matériau métallique contenant du magnésium avec un revêtement inhibant la corrosion contenant des phosphates d'ammonium-magnésium et avec un ciment osseux minéral, dans lequel un matériau métallique contenant du magnésium, dont la surface présente une couche d'oxyde de magnésium et/ou une couche de sel de magnésium, est combiné à un ciment osseux minéral, qui contient des composants poudreux minéraux qui se lient en présence d'eau pour former une matière solide et des sels hydrosolubles contenant des ions phosphate, pour produire un matériau composite solide qui contient le ciment osseux minéral et le matériau métallique contenant du magnésium, dans lequel le matériau métallique contenant du magnésium est mis en contact avec de l'eau avant et/ou pendant la combinaison avec le ciment osseux minéral en présence de sels hydrosolubles contenant des ions ammonium et des ions phosphate et le ciment osseux minéral est mis en contact avec de l'eau pour le lier.

2. Procédé selon la revendication 1, dans lequel le matériau métallique contenant du magnésium est mis en contact avec une solution aqueuse qui contient des sels contenant des ions ammonium et des ions phosphate avant la production du matériau composite.

3. Procédé selon l'une des revendications précédentes, dans lequel une couche qui contient des sels contenant des ions ammonium et des ions phosphate est appliquée sur la surface du matériau métallique contenant du magnésium avant la production du matériau composite et le matériau métallique contenant du magnésium revêtu de sels contenant des ions ammonium et des ions phosphate est mis en contact avec une solution aqueuse avant ou pendant la production du matériau composite.

4. Procédé selon l'une des revendications précédentes, dans lequel le ciment osseux minéral est présent sous la forme d'une pâte anhydre qui contient de la poudre de ciment osseux minéral qui est dispersée dans un liquide porteur anhydre.

5. Procédé selon l'une des revendications précédentes, dans lequel les sels contenant des ions ammonium et des ions phosphate sont des phosphates d'ammonium, de préférence sélectionnés parmi (NH₄)₃PO₄, (NH₄)₂HPO₄, (NH₄)H₂PO₄, des sels d'ammonium de l'acide di-, oligo- et polyphosphorique ainsi que de l'acide phosphorique partiellement substitué, en particulier du glycérophosphate d'ammonium, et de leurs mélanges.

6. Implant osseux comprenant un matériau métallique contenant du magnésium et un ciment osseux minéral qui contient des sels hydrosolubles contenant des ions phosphate, dans lequel le matériau métallique contenant du magnésium présente un revêtement qui contient des phosphates d'ammonium-magnésium.

7. Implant osseux selon la revendication 6, **caractérisé en ce que** la couche contenant les phosphates d'ammonium-magnésium présente une épaisseur de plus de 0,01 µm et de moins de 50 µm.

8. Implant osseux selon la revendication 6 ou 7, **caractérisé en ce qu'**une couche qui contient de l'oxyde de magnésium et/ou des sels de magnésium se trouve entre le matériau métallique contenant du magnésium et la couche de phosphates d'ammonium-magnésium.

9. Implant osseux selon l'une des revendications 6 à 8, dans lequel le matériau métallique contenant du magnésium est présent sous la forme de
- fibres, brins, filaments, fils,
- fils ou brins transformés en tissus, tresses, maillages, tricotages, nappes de fibres, étoffes de nappes de fibres, membranes ou cordons,
- structures planes réalisées à partir de fibres, brins, filaments ou fils,
- tôles perforées ou métal déployé ou corps façonnés à partir de tôles perforées ou de métal déployé ou
- d'une mousse métallique à cellules ouvertes.

10. Nécessaire pour la fabrication d'un implant osseux, contenant
a) une préparation de ciment osseux comprenant une poudre de ciment osseux minéral et des sels hydrosolubles contenant des ions phosphate, et
b) un matériau métallique contenant du magnésium avec une couche d'oxyde de magnésium et/ou une couche de sel de magnésium et des sels contenant des ions ammonium et phosphate, de préférence des phosphates d'ammonium, ou
c) un matériau métallique contenant du magnésium avec un revêtement contenant des phosphates d'ammonium-magnésium et, le cas échéant, des sels contenant des ions ammonium et phosphate.

11. Nécessaire selon la revendication 10, dans lequel la préparation de ciment osseux est présente sous la forme d'une pâte anhydre qui contient de la poudre de ciment osseux minéral dispersée dans un liquide porteur anhydre.

12. Nécessaire selon la revendication 10 ou 11, dans lequel des sels contenant des ions ammonium et phosphate, de préférence des phosphates d'ammonium, sont contenus sous la forme de matières solides ou présents sous la forme d'une solution aqueuse en tant que composant du nécessaire emballé séparément de la préparation de ciment osseux.

13. Nécessaire selon la revendication 10 ou 11, dans lequel des sels contenant des ions ammonium et phosphate, de préférence des phosphates d'ammonium, sont présents sous la forme d'un revêtement sur le matériau métallique contenant du magnésium.

14. Nécessaire selon l'une des revendications 10 à 13, dans lequel le ciment osseux minéral et le matériau métallique contenant du magnésium sont présents sous la forme d'un composant du nécessaire qui est mis en contact avec une solution aqueuse pour la fabrication du matériau composite.

15. Nécessaire selon l'une des revendications 10 à 14, dans lequel le ciment osseux minéral est réalisé sous la forme d'un système à deux composants qui contient un composant anhydre avec de la poudre de ciment osseux minéral et un composant contenant de l'eau.
